# EUROPEAN PATENT APPLICATION

(11) **EP 3 800 201 A1**
(43) Date of publication of application: **07.04.2021**
(21) Application number: 19306248.6
(22) Date of filing: 01.10.2019
(51) Int. Cl.: C07K 16/28, A61P 31/00, A61P 35/00, A61P 37/04

(54) **CD28H STIMULATION ENHANCES NK CELL KILLING ACTIVITIES**

(71) Applicant: INSERM (Institut National de la Santé et de la Recherche Médicale), 75013 Paris (FR); Université de Nantes, 44000 Nantes (FR)
(72) Inventor: HASPOT, Fabienne, 44300 Nantes (FR); DUPLAUYE, Pierre, 44000 Nantes (FR); HUCHET, Virginie, 44450 La Chapelle Basse Mer (FR)
(74) Representative: Icosa

(57) **Abstract**

CD28H (TMIGD2 or IGPR-1) belongs to the CD28 family and is a transmembrane protein with an immunoglobulin-like extracellular domain, a transmembrane domain and a cytoplasmic tail. The main objective of the inventors was to evaluate the consequences of CD28H stimulation on NK with a monoclonal anti-CD28H antibody (clone 4-5) known to activate T cells. The inventors show that circulating CD56bright and CD56dim NK cells express the same level of CD28H. Circulating CD28H+ NK cells are more activated than CD28Hneg, in fact NK CD28H+ cells express more CD69, NKp30 and Nkp46. The agonist anti-CD28H mAb induces a calcium flux in NK cells while the blocking mAb does not. Calcium flux evaluation shows that CD28H synergize with NKP46 but not with CD16. After an overnight stimulation with the agonist anti-CD28H mAb, NK cells express more CD69, Nkp30, NKG2D and less CD16 compared to a control condition with an isotype mAb. This reflects a very strong activation of NK cells. Moreover, the CD28H-primed NK cells treated with the agonistic anti-CD28H mAb secret more granzyme B and more IFN-g in presence of K562. CD28H-primed NK cells increase their cytotoxic capacities against tumor cell lines. When used in vivo in a xeno-GVHD model in NSG-SGM3 mice, the agonistic anti-CD28H mAb increases the frequency of NK cells suggesting a potential role of CD28H in NK homeostasis. Thus CD28H is a strong activator of NK cells and is the potential new therapeutic target for cancer immunotherapy

## Description

### FIELD OF THE INVENTION:

The present invention is in the field of medicine, in particular immunology.

### BACKGROUND OF THE INVENTION:

Cancer immunotherapy with monoclonal antibodies (mAbs) targeting checkpoint inhibitors is able to induce durable responses in multiple types of cancer. Nowadays immunotherapy wishes to combine the release of the brake together with the hit of the gas pedal in a friendlier tumor environment for T cell and/or NK cell attack. New strategies are developed to revive immune responses with either Bispecific T cells engager (i.e. BiTEs) and recently Bi- or Tri- specific Killer cells engager (i.e. BiKEs and TriKEs). Selecting new NK cell engagers are therefore appealing for innovative immunotherapy.

CD28H (TMIGD2 or IGPR-1) belongs to the CD28 family and interacts with HHLA-2, a B7 family's member. CD28H is a transmembrane protein with an immunoglobulin-like extracellular domain, a transmembrane domain and a cytoplasmic tail. CD28H is expressed on naive T Lymphocytes (TLs), NK cells, innate lymphoid cells, plasmacytoid dendritic cells (Crespo et al, 2017) and endothelial cells (Rahimi et al, 2012). While one study described CD28H's activating role on TLs when using an anti-CD28H mAb (Zhu et al, 2013), another study showed that HHLA2-Ig inhibits TLs (Zhao et al, 2013). Recently, CD28H-CAR NK cells, in which CD28H is fused to the cytoplasmic domain of T-cell receptor ζ chain has been shown to trigger lysis of B7H7 tumor cells (Zhuang et al, 2019).

### SUMMARY OF THE INVENTION:

As defined by the claims, the present invention relates to methods and pharmaceutical compositions for enhancing NK cell killing activities.

### DETAILED DESCRIPTION OF THE INVENTION:

One object of the present invention relates to a method of enhancing NK cell killing activities in a subject in need thereof comprising administering to the subject a therapeutically effective amount of a compound capable of stimulating CD28H on NK cells.

As used herein, "NK cells" refers to a sub-population of lymphocytes that is involved in non-conventional immunity. NK cells can be identified by virtue of certain characteristics and biological properties, such as the expression of specific surface antigens including CD56 and/or CD16 for human NK cells, the absence of the alpha/beta or gamma/delta TCR complex on the cell surface, the ability to bind to and kill cells that fail to express "self" MHC/HLA antigens by the activation of specific cytolytic machinery, the ability to kill tumor cells or other diseased cells that express a ligand for NK activating receptors, and the ability to release protein molecules called cytokines that stimulate or inhibit the immune response ("NK cell killing activities"). Any subpopulation of NK cells will also be encompassed by the term NK cells. Within the context of this invention "active" NK cells designate biologically active NK cells, including NK cells having the capacity of lysing target cells or enhancing the immune function of other cells. For instance, an "active" NK cell can be able to kill cells that express a ligand for an activating NK receptor and/or fail to express MHC/HLA antigens recognized by a KIR on the NK cell.

As used herein, the term "CD28H" has its general meaning in the art and refers to a protein that belongs to the CD28 family. CD28H interacts with HHLA-2, a B7 family's member. CD28H is a transmembrane protein with an immunoglobulin-like extracellular domain, a transmembrane domain and a cytoplasmic tail. The term is also known as TMIGD2 or IGPR-1 and H7CR. An exemplary amino acid sequence for CD28H is represented by SEQ ID NO:1. The extracellular domain of CD28H ranges from the amino acid at position 23 to the amino acid at position 150 in SEQ ID NO:1.

SEQ ID NO:1 >sp|Q96BF3|TMIG2_HUMAN Transmembrane and immunoglobulin
domain-containing protein 2 OS=Homo sapiens OX=9606 GN=TMIGD2 PE=1
SV=2
MGSPGMVLGLLVQIWALQEASSLSVQQGPNLLQVRQGSQATLVCQVDQATAWERLRVKWT
KDGAILCQPYITNGSLSLGVCGPQGRLSWQAPSHLTLQLDPVSLNHSGAYVCWAAVEIPE
LEEAEGNITRLFVDPDDPTQNRNRIASFPGFLFVLLGVGSMGVAAIVWGAWFWGRRSCQQ
RDSGNSPGNAFYSNVLYRPRGAPKKSEDCSGEGKDQRGQSIYSTSFPQPAPRQPHLASRP
CPSPRPCPSPRPGHPVSMVRVSPRPSPTQQPRPKGFPKVGEE

As used herein, the expression "compound that is capable of stimulation CD28H on NK cells" refers to any compound that is capable of engaging CD28H (i.e. binding to) and enhancing NK cell killing activities. The ability of the compound of the present invention to enhance NK cell killing activity may be determined by any assay well known in the art. Typically said assay is an in vitro assay wherein NK cells are brought into contact with target cells (e.g. target cells that are recognized and/or lysed by NK cells). For example, the compound can be selected for the ability to increase specific lysis by NK cells by more than about 20%, preferably with at least about 30%, at least about 40%, at least about 50%, or more of the specific lysis obtained at the same effector: target cell ratio with NK cells or NK cell lines that are contacted by the compound of the present invention. Examples of protocols for classical cytotoxicity assays are described, for example, in Pessino et al, J. Exp. Med, 1998, 188 (5): 953-960; Sivori et al, Eur J Immunol, 1999. 29:1656-1666; Brando et al, (2005) J. Leukoc. Biol. 78:359-371; El-Sherbiny et al, (2007) Cancer Research 67(18):8444-9; Nolte-'t Hoen et al, (2007) Blood 109:670-673; Bryceson, Y. T (2006) Blood 107: 159-166); and Vidard, L. (2019) The Journal of Immunology 203 : 676-685. Typically, NK cell cytotoxicity is determined by any assay described in the EXAMPLE. In particular, the compound of the present invention is selected if it causes an increase in the reactivity or cytoxicity of NK cells toward target cells (infected cells, tumor cells, pro-inflammatory cells, etc.), increased activation, activation markers (e.g. CD107 expression, CD69) and/or IFNgamma production in NK cells, and/or increased the frequency in vivo of such activated, reactive, cytotoxic and/or activated NK cells.

In some embodiments, the compound of the present invention is an antibody having specificity to CD28H. More particularly, the antibody binds the extracellular domain of CD28H. More particularly, the antibody binds to an epitope located in the extracellular domain of CD28H.

As used herein the term "antibody" or "immunoglobulin" have the same meaning, and will be used equally in the present invention. The term "antibody" as used herein refers to immunoglobulin molecules and immunologically active portions of immunoglobulin molecules, i.e., molecules that contain an antigen binding site that immunospecifically binds an antigen. As such, the term antibody encompasses not only whole antibody molecules, but also antibody fragments as well as variants (including derivatives) of antibodies and antibody fragments. In natural antibodies, two heavy chains are linked to each other by disulfide bonds and each heavy chain is linked to a light chain by a disulfide bond. There are two types of light chain, lambda (1) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four (α, δ, γ) to five (µ, ε) domains, a variable domain (VH) and three to four constant domains (CH1, CH2, CH3 and CH4 collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from nonhypervariable or framework regions (FR) can participate to the antibody binding site or influence the overall domain structure and hence the combining site. CDRs refer to amino acid sequences which together define the binding affinity and specificity of the natural Fv region of a native immunoglobulin binding site. The light and heavy chains of an immunoglobulin each have three CDRs, designated L-CDR1, L-CDR2, L-CDR3 and H-CDR1, H-CDR2, H-CDR3, respectively. An antigen-binding site, therefore, typically includes six CDRs, comprising the CDR set from each of a heavy and a light chain V region. Framework Regions (FRs) refer to amino acid sequences interposed between CDRs. The residues in antibody variable domains are conventionally numbered according to a system devised by Kabat et al. This system is set forth in Kabat et al., 1987, in Sequences of Proteins of Immunological Interest, US Department of Health and Human Services, NIH, USA (hereafter "Kabat et al."). This numbering system is used in the present specification. The Kabat residue designations do not always correspond directly with the linear numbering of the amino acid residues in SEQ ID sequences. The actual linear amino acid sequence may contain fewer or additional amino acids than in the strict Kabat numbering corresponding to a shortening of, or insertion into, a structural component, whether framework or complementarity determining region (CDR), of the basic variable domain structure. The correct Kabat numbering of residues may be determined for a given antibody by alignment of residues of homology in the sequence of the antibody with a "standard" Kabat numbered sequence. The CDRs of the heavy chain variable domain are located at residues 31-35B (VH-CDR1), residues 50-65 (VH-CDR2) and residues 95-102 (VH-CDR3) according to the Kabat numbering system. The CDRs of the light chain variable domain are located at residues 24-34 (VL-CDR1), residues 50-56 (VL-CDR2) and residues 89-97 (VL-CDR3) according to the Kabat numbering system.

As used herein, the term "specificity" refers to the ability of an antibody to detectably bind an epitope presented on an antigen, such as a CD28H, while having relatively little detectable reactivity with non-CD28H proteins or structures (such as other proteins presented on NK cells, or on other cell types). Specificity can be relatively determined by binding or competitive binding assays, using, e.g., Biacore instruments, as described elsewhere herein. Specificity can be exhibited by, e.g., an about 10:1, about 20:1, about 50:1, about 100:1, 10.000:1 or greater ratio of affinity/avidity in binding to the specific antigen versus nonspecific binding to other irrelevant molecules (in this case the specific antigen is a CD28H polypeptide). The term "affinity", as used herein, means the strength of the binding of an antibody to an epitope. The affinity of an antibody is given by the dissociation constant Kd, defined as [Ab] x [Ag] / [Ab-Ag], where [Ab-Ag] is the molar concentration of the antibody-antigen complex, [Ab] is the molar concentration of the unbound antibody and [Ag] is the molar concentration of the unbound antigen. The affinity constant Ka is defined by 1/Kd. Preferred methods for determining the affinity of mAbs can be found in Harlow, et al., Antibodies: A Laboratory Manual, Cold Spring Harbor Laboratory Press, Cold Spring Harbor, N.Y., 1988), Coligan et al., eds., Current Protocols in Immunology, Greene Publishing Assoc. and Wiley Interscience, N.Y., (1992, 1993), and Muller, Meth. Enzymol. 92:589-601 (1983), which references are entirely incorporated herein by reference. One preferred and standard method well known in the art for determining the affinity of mAbs is the use of Biacore instruments.

The term "affinity", as used herein, means the strength of the binding of an antibody to a target molecule (e.g. an epitope). The affinity of a binding protein is given by the dissociation constant Kd. For an antibody said Kd is defined as [Ab] x [Ag] / [Ab-Ag], where [Ab-Ag] is the molar concentration of the antibody-antigen complex, [Ab] is the molar concentration of the unbound antibody and [Ag] is the molar concentration of the unbound antigen. The affinity constant Ka is defined by 1/Kd. Preferred methods for determining the affinity of a binding protein can be found in Harlow, et al., Antibodies: A Laboratory Manual, Cold Spring Harbor Laboratory Press, Cold Spring Harbor, N.Y., 1988), Coligan et al., eds., Current Protocols in Immunology, Greene Publishing Assoc. and Wiley Interscience, N.Y., (1992, 1993), and Muller, Meth. Enzymol. 92:589-601 (1983), which references are entirely incorporated herein by reference. One preferred and standard method well known in the art for determining the affinity of binding protein is the use of Biacore instruments.

The term "binding" as used herein refers to a direct association between two molecules, due to, for example, covalent, electrostatic, hydrophobic, and ionic and/or hydrogen-bond interactions, including interactions such as salt bridges and water bridges. In particular, as used herein, the term "binding" in the context of the binding of an antibody to a predetermined target molecule (e.g. an antigen or epitope) typically is a binding with an affinity corresponding to a K_{D} of about 10⁻⁷ M or less, such as about 10⁻⁸ M or less, such as about 10⁻⁹ M or less, about 10⁻¹⁰ M or less, or about 10⁻¹¹ M or even less.

As used herein, the term "epitope" refers to a specific arrangement of amino acids located on a protein or proteins to which an antibody binds. Epitopes often consist of a chemically active surface grouping of molecules such as amino acids or sugar side chains, and have specific three-dimensional structural characteristics as well as specific charge characteristics. Epitopes can be linear or conformational, *i.e*., involving two or more sequences of amino acids in various regions of the antigen that may not necessarily be contiguous.

In some embodiments, the antibody of the present invention is a monoclonal antibody. The terms "monoclonal antibody", "monoclonal Ab", "monoclonal antibody composition", "mAb", or the like, as used herein refer to a preparation of antibody molecules of single molecular composition. A monoclonal antibody is obtained from a population of substantially homogeneous antibodies, *i.e*., the individual antibodies comprised in the population are identical except for possible naturally occurring mutations that may be present in minor amounts. A monoclonal antibody composition displays a single binding specificity and affinity for a particular epitope. For instance, monoclonal antibodies may be generated using the method of Kohler and Milstein (Nature, 256:495, 1975). To prepare monoclonal antibodies useful in the invention, a mouse or other appropriate host animal can be immunized at suitable intervals (e.g., twice-weekly, weekly, twice-monthly or monthly) with the appropriate antigenic forms (*i.e*., CD28H polypeptides). The animal may be administered a final "boost" of antigen within one week of sacrifice. It is often desirable to use an immunologic adjuvant during immunization. Suitable immunologic adjuvants include Freund's complete adjuvant, Freund's incomplete adjuvant, alum, Ribi adjuvant, Hunter's Titermax, saponin adjuvants such as QS21 or Quil A, or CpG-containing immunostimulatory oligonucleotides. Other suitable adjuvants are well-known in the field. The animals may be immunized by subcutaneous, intraperitoneal, intramuscular, intravenous, intranasal or other routes. A given animal may be immunized with multiple forms of the antigen by multiple routes. Monoclonal antibodies useful in the invention can also be prepared from non-immunized animals or humans. However, the modifier "monoclonal" is not to be construed as requiring production of the antibody by any particular method. For example, a monoclonal antibody may also be prepared by the hybridoma methodology first described by Kohler et al., Nature, 256:495 (1975), or may be made using recombinant DNA methods in bacterial, eukaryotic animal or plant cells (see, *e.g*., U.S. Pat. No. 4,816,567). A "monoclonal antibody" may also be isolated from phage antibody libraries using the techniques described in Clackson et al., Nature, 352:624-628 (1991) and Marks et al., J. Mol. Biol., 222:581-597 (1991), for example.

In some embodiments, the antibody of the present invention is a chimeric antibody. As used herein, the term "chimeric antibody" refers to an antibody which comprises a VH domain and a VL domain of a non-human antibody, and a CH domain and a CL domain of a human antibody.

In some embodiments, the antibody of the present invention is a humanized antibody. The term "humanized antibody" refers to an antibody having variable region framework and constant regions from a human antibody but retains the CDRs of a previous non-human antibody. In some embodiments, a humanized antibody contains minimal sequence derived from non-human immunoglobulin. For the most part, humanized antibodies and antibody fragments thereof may be human immunoglobulins (recipient antibody or antibody fragment) in which residues from a complementary-determining region (CDR) of the recipient are replaced by residues from a CDR of non-human species (donor antibody) such as mouse, rat or rabbit having the desired specificity, affinity, and capacity.

In some embodiments, the antibody of the present invention is a human antibody. As used herein the term "human monoclonal antibody", is intended to include antibodies having variable and constant regions derived from human immunoglobulin sequences. The human antibodies of the present invention may include amino acid residues not encoded by human immunoglobulin sequences (*e.g*., mutations introduced by random or site-specific mutagenesis *in vitro* or by somatic mutation *in vivo*). More specifically, the term "human monoclonal antibody", as used herein, is not intended to include antibodies in which CDR sequences derived from the germline of another mammalian species, such as a mouse, have been grafted onto human framework sequences.

In some embodiments, the monoclonal antibody of the present invention cross-competes for binding to the CD28H isoform with the antibody comprising a light chain variable region comprising the following CDR: i) the VL-CDR1 as set forth in SEQ ID NO:2 (QSLFSSNTKRNY), ii) the VL-CDR2 as set forth in SEQ ID NO:3 (HAS) and iii) the VL-CDR3 as set forth in SEQ ID NO:4 (GFSITTGGYY) and a heavy chain variable region comprising the following CDR i) the VH-CDR1 as set forth in SEQ ID NO:5 (GYTFTSYW), ii) the VH-CDR2 as set forth in SEQ ID NO:6 (IYTSGRT) and iii) the VH-CDR3 as set forth in SEQ ID NO:7 (ADMADKGGWFAY).

Thus in some embodiments, the antibody of the present invention cross-competes with the monoclonal antibody 4-5 as described in WO2014100823 and in Zhu Y. et al. Nat Commun. 2013;4:2043. Said monoclonal antibody comprises the light chain variable region and the heavy chain variable region as follows:
**Light Chain Variable Region:** FR1-CDR1-FR2-CDR2-FR3-CDR3-FR4 
(SEQ ID NO: 8)
DIVMTQSPSS LAVSAGEKVT ISCLSS QSLF SSNTKRNY LN
WYLQKPGQSP KLLIY HAS TR LTGVPGRFIG SGSGTDFTLT
VSTVQAEDLG DYFC QQHYET PLT FGDGTRL EIK
**Heavy Chain Variable Region:** FR1-CDR1-FR2-CDR2-FR3-CDR3-FR4

(SEQ ID NO: 9)
QIQLQESGPG LVKPSQSLSL TCSVT GFSIT TGGYY WNWIR
QFPGKKLEWM GY IYTSGRT S YNPSLKSRIS ITRDTSKNQF
FLQLNSMTTE DTATYYC ADM ADKGGWFAY W GQGTLVTVSS

As used herein, the term "cross-competes" refers to monoclonal antibodies which share the ability to bind to a specific region of an antigen. In the present disclosure the monoclonal antibody that "cross-competes" has the ability to interfere with the binding of another monoclonal antibody for the antigen in a standard competitive binding assay. Such a monoclonal antibody may, according to non-limiting theory, bind to the same or a related or nearby (e.g., a structurally similar or spatially proximal) epitope as the antibody with which it competes. Cross-competition is present if antibody A reduces binding of antibody B at least by 60%, specifically at least by 70% and more specifically at least by 80% and vice versa in comparison to the positive control which lacks one of said antibodies. As the skilled artisan appreciates competition may be assessed in different assay set-ups. One suitable assay involves the use of the Biacore technology (e.g., by using the BIAcore 3000 instrument (Biacore, Uppsala, Sweden)), which can measure the extent of interactions using surface plasmon resonance technology. Another assay for measuring cross-competition uses an ELISA-based approach. Furthermore, a high throughput process for "binning" antibodies based upon their cross-competition is described in International Patent Application No. WO2003/48731.

Thus, in some embodiments, the antibody of the present invention binds to the same epitope for clone 4-5 as above described.

In some embodiments, the antibody of the present invention comprises a light chain variable region comprising the following CDR: i) the VL-CDR1 as set forth in SEQ ID NO:2 (QSLFSSNTKRNY), ii) the VL-CDR2 as set forth in SEQ ID NO:3 (HAS) and iii) the VL-CDR3 as set forth in SEQ ID NO:4 (GFSITTGGYY) and a heavy chain variable region comprising the following CDR i) the VH-CDR1 as set forth in SEQ ID NO:5 (GYTFTSYW), ii) the VH-CDR2 as set forth in SEQ ID NO:6 (IYTSGRT) and iii) the VH-CDR3 as set forth in SEQ ID NO:7 (ADMADKGGWFAY).

In some embodiments, the antibody of the present invention comprises a light chain variable region as set forth in SEQ ID NO:8 and/or a heavy chain variable region as set forth in SEQ ID NO:9.

In some embodiments, the antibody of the present invention comprises human heavy chain constant regions sequences but will not deplete NK cells to which they are bound and preferably do not comprise an Fc portion that induces antibody dependent cellular cytotoxicity (ADCC). As used herein, the term "depleting", with respect to CD28H-expressing cells means a process, method, or compound that can kill, eliminate, lyse or induce such killing, elimination or lysis, so as to negatively affect the number of CD28H expressing cells present in a sample or in a subject. The terms "Fc domain," "Fc portion," and "Fc region" refer to a C-terminal fragment of an antibody heavy chain, e.g., from about amino acid (aa) 230 to about aa 450 of human gamma heavy chain or its counterpart sequence in other types of antibody heavy chains (e.g., α, δ, ε and µ for human antibodies), or a naturally occurring allotype thereof. Unless otherwise specified, the commonly accepted Kabat amino acid numbering for immunoglobulins is used throughout this disclosure (see Kabat et al. (1991) Sequences of Protein of Immunological Interest, 5th ed., United States Public Health Service, National Institute of Health, Bethesda, MD). In some embodiments the antibody of the present invention does not lead, directly or indirectly, to the depletion of NK cells expressing CD28H polypeptides (e.g. do not lead to a 10%, 20%, 50%, 60% or greater elimination or decrease in number of CD28H+ NK cells). In some embodiments, the antibody of the present invention does not comprise an Fc domain capable of substantially binding to a FcyRIIIA (CD16) polypeptide. In some embodiments, the antibody of the present invention lacks an Fc domain (e.g. lacks a CH2 and/or CH3 domain) or comprises an Fc domain of IgG2 or IgG4 isotype. In some embodiments, the antibody of the present invention consists of or comprises a Fab, Fab', Fab'-SH, F (ab') 2, Fv, a diabody, single-chain antibody fragment, or a multispecific antibody comprising multiple different antibody fragments. In some embodiments, the antibody of the present invention is not linked to a toxic moiety. In some embodiments, one or more amino acids selected from amino acid residues can be replaced with a different amino acid residue such that the antibody has altered C2q binding and/or reduced or abolished complement dependent cytotoxicity (CDC). This approach is described in further detail in U.S. Patent Nos. 6,194,551 by ldusogie et al.

In some embodiments, the antibody of the present invention an antibody fragment. As used herein, the term "antibody fragment" refers to at least one portion of an intact antibody, preferably the antigen binding region or variable region of the intact antibody, that retains the ability to specifically interact with (*e.g*., by binding, steric hindrance, stabilizing/destabilizing, spatial distribution) an epitope of an antigen. Examples of antibody fragments include, but are not limited to, Fab, Fab', F(ab')₂, Fv fragments, single chain antibody molecules, in particular scFv antibody fragments, disulfide-linked Fvs (sdFv), a Fd fragment consisting of the VH and CHI domains, linear antibodies, single domain antibodies such as, for example, sdAb (either VL or VH), camelid VHH domains, multi-specific antibodies formed from antibody fragments such as, for example, a bivalent fragment comprising two Fab fragments linked by a disulfide bridge at the hinge region, and an isolated CDR or other epitope binding fragments of an antibody. An antigen binding fragment can also be incorporated into single domain antibodies, maxibodies, minibodies, nanobodies, intrabodies, diabodies, triabodies, tetrabodies, v-NAR and bis-scFv (see, *e.g*., Hollinger and Hudson, Nature Biotechnology 23:1126-1136, 2005). Antigen binding fragments can also be grafted into scaffolds based on polypeptides such as a fibronectin type III (see U.S. Patent No.: 6,703,199, which describes fibronectin polypeptide minibodies). Papain digestion of antibodies produces two identical antigen-binding fragments, called "Fab" fragments, and a residual "Fc" fragment, a designation reflecting the ability to crystallize readily.

Fragments and derivatives of antibodies of this invention (which are encompassed by the term "antibody" as used in this application, unless otherwise stated or clearly contradicted by context), can be produced by techniques that are known in the art. "Fragments" comprise a portion of the intact antibody, generally the antigen binding site or variable region. Examples of antibody fragments include Fab, Fab', Fab'-SH, F(ab')2, and Fv fragments; diabodies; any antibody fragment that is a polypeptide having a primary structure consisting of one uninterrupted sequence of contiguous amino acid residues (referred to herein as a "single-chain antibody fragment" or "single chain polypeptide"), including without limitation (1) single - chain Fv molecules (2) single chain polypeptides containing only one light chain variable domain, or a fragment thereof that contains the three CDRs of the light chain variable domain, without an associated heavy chain moiety and (3) single chain polypeptides containing only one heavy chain variable region, or a fragment thereof containing the three CDRs of the heavy chain variable region, without an associated light chain moiety; and multispecific antibodies formed from antibody fragments. Fragments of the present antibodies can be obtained using standard methods.

For instance, Fab or F(ab')₂ fragments may be produced by protease digestion of the isolated antibodies, according to conventional techniques. It will be appreciated that immunoreactive fragments can be modified using known methods, for example to slow clearance *in vivo* and obtain a more desirable pharmacokinetic profile the fragment may be modified with polyethylene glycol (PEG). Methods for coupling and site-specifically conjugating PEG to a Fab' fragment are described in, for example, Leong et al., Cytokines 16 (3): 106-119 (2001) and Delgado et al., Br. J. Cancer 5 73 (2): 175- 182 (1996), the disclosures of which are incorporated herein by reference.

In some embodiments, the antibody is a nanobody. As used herein the term "nanobody" has its general meaning in the art and refers to an antibody-derived therapeutic protein that contains the unique structural and functional properties of naturally-occurring heavy chain antibodies. These heavy chain antibodies contain a single variable domain (VHH) and two constant domains (CH2 and CH3). As used herein, the term "derived" indicates a relationship between a first and a second molecule. It generally refers to structural similarity between the first molecule and the second molecule and does not connote or include a process or source limitation on a first molecule that is derived from a second molecule.

In some embodiments, the present invention provides a multispecific antibody comprising a first antigen binding site from an antibody of the present invention herein above and at least one second antigen binding site. Accordingly, the first antigen-binding site is used for recruiting a killing mechanism, i.e. increasing NK cytoxicity. Typically, the second antigen-binding site binds to an antigen that is expressed by a cancer cell or a cell infected by a virus, bacteria... In some embodiments, the second antigen-binding site binds to an antigen on a human B cell, such as, e.g., CD19, CD20, CD21, CD22, CD23, CD80, CD138 and HLA-DR. In some embodiments, the second antigen-binding site binds to an antigen which is a tumor-associated antigen (TAA). Exemplary TAAs include carcinoembryonic antigen (CEA), prostate specific antigen (PSA), RAGE (renal antigen), α-fetoprotein, CAMEL (CTL-recognized antigen on melanoma), CT antigens (such as MAGE-B5, -B6, -C2, -C3, and D; Mage-12; CT10; NY-ESO-1, SSX-2, GAGE, BAGE, MAGE, and SAGE), mucin antigens (e.g., MUC1, mucin-CA125, etc.), ganglioside antigens, tyrosinase, gp75, c-Met, Marti, MelanA, MUM-1, MUM-2, MUM-3, HLA-B7, Ep-CAM or a cancer-associated integrin, such as α5β3 integrin. Exemplary formats for the multispecific antibody molecules of the present invention include, but are not limited to (i) two antibodies cross-linked by chemical heteroconjugation, one with a specificity to CD28H and another with a specificity to a second antigen; (ii) a single antibody that comprises two different antigen-binding regions; (iii) a single-chain antibody that comprises two different antigen-binding regions, e.g., two scFvs linked in tandem by an extra peptide linker; (iv) a dual-variable-domain antibody (DVD-Ig), where each light chain and heavy chain contains two variable domains in tandem through a short peptide linkage (Wu et al., Generation and Characterization of a Dual Variable Domain Immunoglobulin (DVD-Ig™) Molecule, In : Antibody Engineering, Springer Berlin Heidelberg (2010)); (v) a chemically-linked bispecific (Fab')2 fragment; (vi) a Tandab, which is a fusion of two single chain diabodies resulting in a tetravalent bispecific antibody that has two binding sites for each of the target antigens; (vii) a flexibody, which is a combination of scFvs with a diabody resulting in a multivalent molecule; (viii) a so called "dock and lock" molecule, based on the "dimerization and docking domain" in Protein Kinase A, which, when applied to Fabs, can yield a trivaient bispecific binding protein consisting of two identical Fab fragments linked to a different Fab fragment; (ix) a so-called Scorpion molecule, comprising, e.g., two scFvs fused to both termini of a human Fab-arm; and (x) a diabody. Another exemplary format for bispecific antibodies is IgG-like molecules with complementary CH3 domains to force heterodimerization. Such molecules can be prepared using known technologies, such as, e.g., those known as Triomab/Quadroma (Trion Pharma/Fresenius Biotech), Knob-into-Hole (Genentech), CrossMAb (Roche) and electrostatically-matched (Amgen), LUZ-Y (Genentech), Strand Exchange Engineered Domain body (SEEDbody)(EMD Serono), Biclonic (Merus) and DuoBody (Genmab A/S) technologies. In some embodiments, the bispecific antibody is obtained or obtainable via a controlled Fab-arm exchange, typically using DuoBody technology. In vitro methods for producing bispecific antibodies by controlled Fab-arm exchange have been described in WO2008119353 and WO 2011131746 (both by Genmab A/S). In one exemplary method, described in WO 2008119353, a bispecific antibody is formed by "Fab-arm" or "half- molecule" exchange (swapping of a heavy chain and attached light chain) between two monospecific antibodies, both comprising IgG4-like CH3 regions, upon incubation under reducing conditions. The resulting product is a bispecific antibody having two Fab arms which may comprise different sequences. In another exemplary method, described in WO 2011131746, bispecific antibodies of the present invention are prepared by a method comprising the following steps, wherein at least one of the first and second antibodies is a antibody of the present invention : a) providing a first antibody comprising an Fc region of an immunoglobulin, said Fc region comprising a first CH3 region; b) providing a second antibody comprising an Fc region of an immunoglobulin, said Fc region comprising a second CH3 region; wherein the sequences of said first and second CH3 regions are different and are such that the heterodimeric interaction between said first and second CH3 regions is stronger than each of the homodimeric interactions of said first and second CH3 regions; c) incubating said first antibody together with said second antibody under reducing conditions; and d) obtaining said bispecific antibody, wherein the first antibody is a antibody of the present invention and the second antibody has a different binding specificity, or vice versa. The reducing conditions may, for example, be provided by adding a reducing agent, e.g. selected from 2-mercaptoethylamine, dithiothreitol and tris(2-carboxyethyl)phosphine. Step d) may further comprise restoring the conditions to become non-reducing or less reducing, for example by removal of a reducing agent, e.g. by desalting. Preferably, the sequences of the first and second CH3 regions are different, comprising only a few, fairly conservative, asymmetrical mutations, such that the heterodimeric interaction between said first and second CH3 regions is stronger than each of the homodimeric interactions of said first and second CH3 regions. More details on these interactions and how they can be achieved are provided in WO 2011131746, which is hereby incorporated by reference in its entirety.

The antibody of the present invention may be produced by any technique known in the art, such as, without limitation, any chemical, biological, genetic or enzymatic technique, either alone or in combination. For example, knowing the amino acid sequence of the desired sequence, one skilled in the art can readily produce said antibodies, by standard techniques for production of polypeptides. For instance, they can be synthesized using well-known solid phase method, preferably using a commercially available peptide synthesis apparatus (such as that made by Applied Biosystems, Foster City, California) and following the manufacturer' s instructions. Alternatively, antibodies of the present invention can be synthesized by recombinant DNA techniques well-known in the art. For example, antibodies can be obtained as DNA expression products after incorporation of DNA sequences encoding the antibodies into expression vectors and introduction of such vectors into suitable eukaryotic or prokaryotic hosts that will express the desired antibodies, from which they can be later isolated using well-known techniques.

In some embodiments, the subject suffers from a cancer or an infectious disease. Accordingly, a further object of the present invention relates to a method of treating a cancer or an infectious disease in a subject in need thereof comprising administering to the subject a therapeutically effective amount of a compound capable of stimulating CD28H on NK cells.

As used herein, "treatment" or "treating" is an approach for obtaining beneficial or desired results including clinical results. For purposes of this invention, beneficial or desired clinical results include, but are not limited to, one or more of the following: alleviating one or more symptoms resulting from the disease, diminishing the extent of the disease, stabilizing the disease (*e.g*., preventing or delaying the worsening of the disease), preventing or delaying the spread (*e.g*., metastasis) of the disease, preventing or delaying the recurrence of the disease, delay or slowing the progression of the disease, ameliorating the disease state, providing a remission (partial or total) of the disease, decreasing the dose of one or more other medications required to treat the disease, delaying the progression of the disease, increasing the quality of life, and/or prolonging survival. Also encompassed by "treatment" is a reduction of pathological consequence of cancer. The methods of the present invention contemplate any one or more of these aspects of treatment. In some embodiments, the terms "treating" or "treatment" refers to both therapeutic treatment and prophylactic or preventative measures; wherein the object is to prevent or slow down (lessen) the targeted disease. Therefore, in some embodiments, those in need of treatment may include those already with the disorder as well as those prone to have the disorder or those in whom the disorder is to be prevented.

As used herein, the term "cancer" has its general meaning in the art and includes, but is not limited to, solid tumors and blood borne tumors The term cancer includes diseases of the skin, tissues, organs, bone, cartilage, blood and vessels. The term "cancer" further encompasses both primary and metastatic cancers. Examples of cancers that may treated by methods and compositions of the invention include, but are not limited to, cancer cells from the bladder, blood, bone, bone marrow, brain, breast, colon, esophagus, gastrointestinal, gum, head, kidney, liver, lung, nasopharynx, neck, ovary, prostate, skin, stomach, testis, tongue, or uterus. In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous; adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; and roblastoma, malignant; Sertoli cell carcinoma; leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; malign melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; Hodgkin's disease; Hodgkin's lymphoma; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia.

As used herein the term "infectious disease" includes any infection caused by viruses, bacteria, protozoa, molds or fungi. In some embodiments, the viral infection comprises infection by one or more viruses selected from the group consisting of *Arenaviridae*, *Astroviridae*, *Birnaviridae*, *Bromoviridae, Bunyaviridae, Caliciviridae, Closteroviridae, Comoviridae, Cystoviridae, Flaviviridae, Flexiviridae, Hepevirus, Leviviridae, Luteoviridae, Mononegavirales, Mosaic Viruses, Nidovirales, Nodaviridae, Orthomyxoviridae, Picobirnavirus, Picornaviridae, Potyviridae, Reoviridae, Retroviridae, Sequiviridae, Tenuivirus, Togaviridae, Tombusviridae, Totiviridae, Tymoviridae, Hepadnaviridae, Herpesviridae, Paramyxoviridae or Papillomaviridae viruses. Relevant taxonomic families of RNA viruses include, without limitation, Astroviridae, Birnaviridae, Bromoviridae, Caliciviridae, Closteroviridae, Comoviridae, Cystoviridae, Flaviviridae, Flexiviridae, Hepevirus, Leviviridae, Luteoviridae, Mononegavirales, Mosaic Viruses, Nidovirales, Nodaviridae, Orthomyxoviridae, Picobirnavirus, Picornaviridae, Potyviridae, Reoviridae, Retroviridae, Sequiviridae, Tenuivirus, Togaviridae, Tombusviridae, Totiviridae,* and *Tymoviridae* viruses. In some embodiments, the viral infection comprises infection by one or more viruses selected from the group consisting of adenovirus, rhinovirus, hepatitis, immunodeficiency virus, polio, measles, Ebola, Coxsackie, Rhino, West Nile, small pox, encephalitis, yellow fever, Dengue fever, influenza (including human, avian, and swine), lassa, lymphocytic choriomeningitis, junin, machuppo, guanarito, hantavirus, Rift Valley Fever, La Crosse, California encephalitis, Crimean-Congo, Marburg, Japanese Encephalitis, Kyasanur Forest, Venezuelan equine encephalitis, Eastern equine encephalitis, Western equine encephalitis, severe acute respiratory syndrome (SARS), parainfluenza, respiratory syncytial, Punta Toro, Tacaribe, pachindae viruses, adenovirus, Dengue fever, influenza A and influenza B (including human, avian, and swine), junin, measles, parainfluenza, Pichinde, punta toro, respiratory syncytial, rhinovirus, Rift Valley Fever, severe acute respiratory syndrome (SARS), Tacaribe, Venezuelan equine encephalitis, West Nile and yellow fever viruses, tick-borne encephalitis virus, Japanese encephalitis virus, St. Louis encephalitis virus, Murray Valley virus, Powassan virus, Rocio virus, louping-ill virus, Banzi virus, Ilheus virus, Kokobera virus, Kunjin virus, Alfuy virus, bovine diarrhea virus, and Kyasanur forest disease. Bacterial infections that can be treated according to this invention include, but are not limited to, infections caused by the following: *Staphylococcus; Streptococcus,* including *S. pyogenes; Enterococcl; Bacillus,* including *Bacillus anthracis,* and *Lactobacillus; Listeria; Corynebacterium diphtheriae; Gardnerella* including G. *vaginalis; Nocardia; Streptomyces; Thermoactinomyces vulgaris; Treponerna; Camplyobacter, Pseudomonas* including *aeruginosa; Legionella; Neisseria including N.gonorrhoeae and N.meningitides; Flavobacterium* including *F. meningosepticum and F. odoraturn; Brucella; Bordetella* including *B. pertussis and B. bronchiseptica; Escherichia* including *E. coli, Klebsiella; Enterobacter, Serratia including S. marcescens* and *S*. *liquefaciens; Edwardsiella; Proteus* including *P. mirabilis* and *P. vulgaris; Streptobacillus; Rickettsiaceae* including R. *fickettsfi, Chlamydia* including *C. psittaci and C. trachornatis; Mycobacterium* including *M. tuberculosis, M. intracellulare, M. folluiturn, M. laprae, M. avium, M. bovis, M. africanum, M. kansasii, M. intracellulare,* and *M. lepraernurium; and Nocardia.* Protozoa infections that may be treated according to this invention include, but are not limited to, infections caused by leishmania, kokzidioa, and trypanosoma. A complete list of infectious diseases can be found on the website of the National Center for Infectious Disease (NCID) at the Center for Disease Control (CDC) (World Wide Web (www) at cdc.gov/ncidod/diseases/), which list is incorporated herein by reference. All of said diseases are candidates for treatment using the compositions according to the invention.

As used herein, the term "therapeutically effective amount" refers to an amount effective, at dosages and for periods of time necessary, to achieve a desired therapeutic result. A therapeutically effective amount of the compound of the present invention may vary according to factors such as the disease state, age, sex, and weight of the individual, and the ability of the compound of the present invention to elicit a desired response in the individual. A therapeutically effective amount is also one in which any toxic or detrimental effects of the antibody or antibody portion are outweighed by the therapeutically beneficial effects. The efficient dosages and dosage regimens for the compound of the present invention depend on the disease or condition to be treated and may be determined by the persons skilled in the art. A physician having ordinary skill in the art may readily determine and prescribe the effective amount of the pharmaceutical composition required. For example, the physician could start doses of the compound of the present invention employed in the pharmaceutical composition at levels lower than that required achieving the desired therapeutic effect and gradually increasing the dosage until the desired effect is achieved. In general, a suitable dose of a composition of the present invention will be that amount of the compound, which is the lowest dose effective to produce a therapeutic effect according to a particular dosage regimen. Such an effective dose will generally depend upon the factors described above. For example, a therapeutically effective amount for therapeutic use may be measured by its ability to stabilize the progression of disease. Typically, the ability of a compound to inhibit cancer may, for example, be evaluated in an animal model system predictive of efficacy in human tumors. Alternatively, this property of a composition may be evaluated by examining the ability of the compound to induce cytotoxicity by in vitro assays known to the skilled practitioner. A therapeutically effective amount of a therapeutic compound may decrease tumor size, or otherwise ameliorate symptoms in a subject. One of ordinary skill in the art would be able to determine such amounts based on such factors as the subject's size, the severity of the subject's symptoms, and the particular composition or route of administration selected. An exemplary, non-limiting range for a therapeutically effective amount of a compound of the present invention is about 0.1-100 mg/kg, such as about 0.1-50 mg/kg, for example about 0.1-20 mg/kg, such as about 0.1-10 mg/kg, for instance about 0.5, about such as 0.3, about 1, about 3 mg/kg, about 5 mg/kg or about 8 mg/kg. An exemplary, non-limiting range for a therapeutically effective amount of a compound of the present invention is 0.02-100 mg/kg, such as about 0.02-30 mg/kg, such as about 0.05-10 mg/kg or 0.1-3 mg/kg, for example about 0.5-2 mg/kg. Administration may e.g. be intravenous, intramuscular, intraperitoneal, or subcutaneous, and for instance administered proximal to the site of the target. Dosage regimens in the above methods of treatment and uses are adjusted to provide the optimum desired response (e.g., a therapeutic response). For example, a single bolus may be administered, several divided doses may be administered over time or the dose may be proportionally reduced or increased as indicated by the exigencies of the therapeutic situation. In some embodiments, the efficacy of the treatment is monitored during the therapy, e.g. at predefined points in time. In some embodiments, the efficacy may be monitored by visualization of the disease area, or by other diagnostic methods described further herein, e.g. by performing one or more PET-CT scans, for example using a labeled compound of the present invention, fragment or mini-antibody derived from the compound of the present invention. If desired, an effective daily dose of a pharmaceutical composition may be administered as two, three, four, five, six or more sub-doses administered separately at appropriate intervals throughout the day, optionally, in unit dosage forms. In some embodiments, the human monoclonal antibodies of the present invention are administered by slow continuous infusion over a long period, such as more than 24 hours, in order to minimize any unwanted side effects. An effective dose of a compound of the present invention may also be administered using a weekly, biweekly or triweekly dosing period. The dosing period may be restricted to, e.g., 8 weeks, 12 weeks or until clinical progression has been established. As non-limiting examples, treatment according to the present invention may be provided as a daily dosage of a compound of the present invention in an amount of about 0.1-100 mg/kg, such as 0.2, 0.5, 0.9, 1.0, 1.1, 1.5, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 45, 50, 60, 70, 80, 90 or 100 mg/kg, per day, on at least one of days 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40, or alternatively, at least one of weeks 1, 2, 3, 4, 5, 6, 7, 8, 9, 10, 11, 12, 13, 14, 15, 16, 17, 18, 19 or 20 after initiation of treatment, or any combination thereof, using single or divided doses every 24, 12, 8, 6, 4, or 2 hours, or any combination thereof.

The present invention also provides for therapeutic applications where a compound of the present invention is used in combination with at least one further therapeutic agent, e.g. for treating cancer. Such administration may be simultaneous, separate or sequential. For simultaneous administration the agents may be administered as one composition or as separate compositions, as appropriate. The further therapeutic agent is typically relevant for the disorder to be treated. Exemplary therapeutic agents include other anti-cancer antibodies, cytotoxic agents, chemotherapeutic agents, anti-angiogenic agents, anti-cancer immunogens, cell cycle control/apoptosis regulating agents, hormonal regulating agents, and other agents described below.

In some embodiments, the second agent is a natural ligand of an NK cell activating or an antibody that binds and activates an NK cell activating receptor other than CD28H. In some embodiments, the agent is an agent that increases the presence of a natural ligand of an NK cell activating receptor on the surface of an target cell (e.g., infected cells, or tumor cells). NK cell activating receptors include, for example, NKG2D or activating KIR receptors (KIR2DS receptors, KIR2DS2, KIR2DS4). As used herein, the term "activating NK receptor" refers to any molecule on the surface of NK cells that, when stimulated, causes a measurable increase in any property or activity known in the art as associated with NK activity, such as cytokine (for example IFN-γ and TNF-α) production, increases in intracellular free calcium levels, the ability to target cells in a redirected killing assay as described, e.g. elsewhere in the present specification, or the ability to stimulate NK cell proliferation. The term "activating NK receptor" includes but is not limited to activating forms or KIR proteins (for example KIR2DS proteins), NKG2D, IL-2R, IL-12R, IL-15R, IL-18R and IL-21R. Examples of ligands that act as agonists at activating receptors include, e.g. IL-2, IL-15, IL-21 polypeptides. In some embodiments, the second antibody is specific for CD137. As used herein the term "CD137" has its general meaning in the art and may also be referred to as Ly63, ILA or 4-1BB. CD137 is a member of the tumor necrosis factor (TNF) receptor family. Members of this receptor family and their structurally related ligands are important regulators of a wide variety of physiologic processes and play an important role in the regulation of immune responses. CD137 is expressed by activated NK cells, T and B lymphocytes and monocytes/macrophages. The gene encodes a 255-amino acid protein with 3 cysteine-rich motifs in the extracellular domain (characteristic of this receptor family), a transmembrane region, and a short N- terminal cytoplasmic portion containing potential phosphorylation sites. Expression in primary cells is strictly activation dependent. The ligand for the receptor is TNFSF9. Human CD137 is reported to bind only to its ligand. Agonists include the native ligand (TNFSF9), aptamers (see McNamara et al. (2008) J. Clin. Invest. 1 18: 376-386), and antibodies.

In some embodiments, the compound of the present invention is used in combination with a chemotherapeutic agent. The term "chemotherapeutic agent" refers to chemical compounds that are effective in inhibiting tumor growth. Examples of chemotherapeutic agents include alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; acetogenins (especially bullatacin and bullatacinone); a carnptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estrarnustine, ifosfamide, mechlorethamine, mechlorethamine oxide hydrochloride, melphalan, novembichin, phenesterine, prednimus tine, trofosfamide, uracil mustard; nitrosureas such as carmustine, chlorozotocin, fotemustine, lomustine, nimustine, ranimustine; antibiotics such as the enediyne antibiotics (e.g. calicheamicin, especially calicheamicin (11 and calicheamicin 211, see, e.g., Agnew Chem Intl. Ed. Engl. 33:183-186 (1994); dynemicin, including dynemicin A; an esperamicin; as well as neocarzinostatin chromophore and related chromoprotein enediyne antiobiotic chromomophores), aclacinomysins, actinomycin, authramycin, azaserine, bleomycins, cactinomycin, carabicin, canninomycin, carzinophilin, chromomycins, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idanrbicin, marcellomycin, mitomycins, mycophenolic acid, nogalarnycin, olivomycins, peplomycin, potfiromycin, puromycin, quelamycin, rodorubicin, streptomgrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pento statin; phenamet; pirarubicin; podophyllinic acid; 2-ethylhydrazide; procarbazine; PSK®; razoxane; rhizoxin; sizofiran; spirogennanium; tenuazonic acid; triaziquone; 2,2',2"-trichlorotriethylarnine; trichothecenes (especially T-2 toxin, verracurin A, roridinA and anguidine); urethan; vindesine; dacarbazine; mannomustine; mitobromtol; mitolactol; pipobroman; gacytosine; arabinoside ("Ara-C"); cyclophosphamide; thiotepa; taxoids, e.g. paclitaxel (TAXOL®, Bristol-Myers Squibb Oncology, Princeton, N.].) and doxetaxel (TAXOTERE®, Rhone-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-1 1 ; topoisomerase inhibitor RFS 2000; difluoromethylornithine (DMFO); retinoic acid; capecitabine; and phannaceutically acceptable salts, acids or derivatives of any of the above. Also included in this definition are antihormonal agents that act to regulate or inhibit honnone action on tumors such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and phannaceutically acceptable salts, acids or derivatives of any of the above.

In some embodiments, the compound of the present invention is used in combination with a targeted cancer therapy. Targeted cancer therapies are drugs or other substances that block the growth and spread of cancer by interfering with specific molecules ("molecular targets") that are involved in the growth, progression, and spread of cancer. Targeted cancer therapies are sometimes called "molecularly targeted drugs," "molecularly targeted therapies," "precision medicines," or similar names. In some embodiments, the targeted therapy consists of administering the subject with a tyrosine kinase inhibitor. The term "tyrosine kinase inhibitor" refers to any of a variety of therapeutic agents or drugs that act as selective or non-selective inhibitors of receptor and/or non-receptor tyrosine kinases. Tyrosine kinase inhibitors and related compounds are well known in the art and described in U.S Patent Publication 2007/0254295, which is incorporated by reference herein in its entirety. It will be appreciated by one of skill in the art that a compound related to a tyrosine kinase inhibitor will recapitulate the effect of the tyrosine kinase inhibitor, e.g., the related compound will act on a different member of the tyrosine kinase signaling pathway to produce the same effect as would a tyrosine kinase inhibitor of that tyrosine kinase. Examples of tyrosine kinase inhibitors and related compounds suitable for use in methods of embodiments of the present invention include, but are not limited to, dasatinib (BMS-354825), PP2, BEZ235, saracatinib, gefitinib (Iressa), sunitinib (Sutent; SU11248), erlotinib (Tarceva; OSI-1774), lapatinib (GW572016; GW2016), canertinib (CI 1033), semaxinib (SU5416), vatalanib (PTK787/ZK222584), sorafenib (BAY 43-9006), imatinib (Gleevec; STI571), leflunomide (SU101), vandetanib (Zactima; ZD6474), MK-2206 (8-[4-aminocyclobutyl)phenyll-9-phenyl-1,2,4-triazolo[3,4-f][1,6]naphthyridin-3(2H)-one hydrochloride) derivatives thereof, analogs thereof, and combinations thereof. Additional tyrosine kinase inhibitors and related compounds suitable for use in the present invention are described in, for example, U.S Patent Publication 2007/0254295, U.S. Pat. Nos. 5,618,829, 5,639,757, 5,728,868, 5,804,396, 6,100,254, 6,127,374, 6,245,759, 6,306,874, 6,313,138, 6,316,444, 6,329,380, 6,344,459, 6,420,382, 6,479,512, 6,498,165, 6,544,988, 6,562,818, 6,586,423, 6,586,424, 6,740,665, 6,794,393, 6,875,767, 6,927,293, and 6,958,340, all of which are incorporated by reference herein in their entirety. In some embodiments, the tyrosine kinase inhibitor is a small molecule kinase inhibitor that has been orally administered and that has been the subject of at least one Phase I clinical trial, more preferably at least one Phase II clinical, even more preferably at least one Phase III clinical trial, and most preferably approved by the FDA for at least one hematological or oncological indication. Examples of such inhibitors include, but are not limited to, Gefitinib, Erlotinib, Lapatinib, Canertinib, BMS-599626 (AC-480), Neratinib, KRN-633, CEP-11981, Imatinib, Nilotinib, Dasatinib, AZM-475271, CP-724714, TAK-165, Sunitinib, Vatalanib, CP-547632, Vandetanib, Bosutinib, Lestaurtinib, Tandutinib, Midostaurin, Enzastaurin, AEE-788, Pazopanib, Axitinib, Motasenib, OSI-930, Cediranib, KRN-951, Dovitinib, Seliciclib, SNS-032, PD-0332991, MKC-I (Ro-317453; R-440), Sorafenib, ABT-869, Brivanib (BMS-582664), SU-14813, Telatinib, SU-6668, (TSU-68), L-21649, MLN-8054, AEW-541, and PD-0325901.

In some embodiments, the compound of the present invention is used in combination with an immunotherapeutic agent. The term "immunotherapeutic agent," as used herein, refers to a compound, composition or treatment that indirectly or directly enhances, stimulates or increases the body's immune response against cancer cells and/or that decreases the side effects of other anticancer therapies. Immunotherapy is thus a therapy that directly or indirectly stimulates or enhances the immune system's responses to cancer cells and/or lessens the side effects that may have been caused by other anti-cancer agents. Immunotherapy is also referred to in the art as immunologic therapy, biological therapy biological response modifier therapy and biotherapy. Examples of common immunotherapeutic agents known in the art include, but are not limited to, cytokines, cancer vaccines, monoclonal antibodies and non-cytokine adjuvants. Alternatively the immunotherapeutic treatment may consist of administering the subject with an amount of immune cells (T cells, NK, cells, dendritic cells, B cells...). Immunotherapeutic agents can be non-specific, i.e. boost the immune system generally so that the human body becomes more effective in fighting the growth and/or spread of cancer cells, or they can be specific, i.e. targeted to the cancer cells themselves immunotherapy regimens may combine the use of non-specific and specific immunotherapeutic agents. Non-specific immunotherapeutic agents are substances that stimulate or indirectly improve the immune system. Non-specific immunotherapeutic agents have been used alone as a main therapy for the treatment of cancer, as well as in addition to a main therapy, in which case the non-specific immunotherapeutic agent functions as an adjuvant to enhance the effectiveness of other therapies (e.g. cancer vaccines). Non-specific immunotherapeutic agents can also function in this latter context to reduce the side effects of other therapies, for example, bone marrow suppression induced by certain chemotherapeutic agents. Non-specific immunotherapeutic agents can act on key immune system cells and cause secondary responses, such as increased production of cytokines and immunoglobulins. Alternatively, the agents can themselves comprise cytokines. Non-specific immunotherapeutic agents are generally classified as cytokines or non-cytokine adjuvants. A number of cytokines have found application in the treatment of cancer either as general non-specific immunotherapies designed to boost the immune system, or as adjuvants provided with other therapies. Suitable cytokines include, but are not limited to, interferons, interleukins and colony-stimulating factors. Interferons (IFNs) contemplated by the present invention include the common types of IFNs, IFN-alpha (IFN-α), IFN-beta (IFN-β) and IFN-gamma (IFN-γ). IFNs can act directly on cancer cells, for example, by slowing their growth, promoting their development into cells with more normal behavior and/or increasing their production of antigens thus making the cancer cells easier for the immune system to recognise and destroy. IFNs can also act indirectly on cancer cells, for example, by slowing down angiogenesis, boosting the immune system and/or stimulating natural killer (NK) cells, T cells and macrophages. Recombinant IFN-alpha is available commercially as Roferon (Roche Pharmaceuticals) and Intron A (Schering Corporation). Interleukins contemplated by the present invention include IL-2, IL-4, IL-11 and IL-12. Examples of commercially available recombinant interleukins include Proleukin® (IL-2; Chiron Corporation) and Neumega® (IL-12; Wyeth Pharmaceuticals). Zymogenetics, Inc. (Seattle, Wash.) is currently testing a recombinant form of IL-21, which is also contemplated for use in the combinations of the present invention. Colony-stimulating factors (CSFs) contemplated by the present invention include granulocyte colony stimulating factor (G-CSF or filgrastim), granulocyte-macrophage colony stimulating factor (GM-CSF or sargramostim) and erythropoietin (epoetin alfa, darbepoietin). Treatment with one or more growth factors can help to stimulate the generation of new blood cells in subjects undergoing traditional chemotherapy. Accordingly, treatment with CSFs can be helpful in decreasing the side effects associated with chemotherapy and can allow for higher doses of chemotherapeutic agents to be used. Various-recombinant colony stimulating factors are available commercially, for example, Neupogen® (G-CSF; Amgen), Neulasta (pelfilgrastim; Amgen), Leukine (GM-CSF; Berlex), Procrit (erythropoietin; Ortho Biotech), Epogen (erythropoietin; Amgen), Arnesp (erytropoietin). Combination compositions and combination administration methods of the present invention may also involve "whole cell" and "adoptive" immunotherapy methods. For instance, such methods may comprise infusion or re-infusion of immune system cells (for instance tumor-infiltrating lymphocytes (TILs), such as CC2+ and/or CD8+ T cells (for instance T cells expanded with tumor-specific antigens and/or genetic enhancements), antibody-expressing B cells or other antibody-producing or -presenting cells, dendritic cells (e.g., dendritic cells cultured with a DC-expanding agent such as GM-CSF and/or Flt3-L, and/or tumor-associated antigen-loaded dendritic cells), anti-tumor NK cells, so-called hybrid cells, or combinations thereof. Cell lysates may also be useful in such methods and compositions. Cellular "vaccines" in clinical trials that may be useful in such aspects include Canvaxin™, APC-8015 (Dendreon), HSPPC-96 (Antigenics), and Melacine® cell lysates. Antigens shed from cancer cells, and mixtures thereof (see for instance Bystryn et al., Clinical Cancer Research Vol. 7, 1882-1887, July 2001), optionally admixed with adjuvants such as alum, may also be components in such methods and combination compositions.

In some embodiments, the compound of the present invention is used in combination with radiotherapy. Radiotherapy may comprise radiation or associated administration of radiopharmaceuticals to a patient. The source of radiation may be either external or internal to the patient being treated (radiation treatment may, for example, be in the form of external beam radiation therapy (EBRT) or brachytherapy (BT)). Radioactive elements that may be used in practicing such methods include, e.g., radium, cesium-137, iridium-192, americium-241, gold-198, cobalt-57, copper-67, technetium-99, iodide-123, iodide-131, and indium-111.

In some embodiments, the compound of the present invention is used in combination with an immune checkpoint inhibitor.

As used herein, the term "immune checkpoint inhibitor" has its general meaning in the art and refers to any compound inhibiting the function of an immune inhibitory checkpoint protein. As used herein the term "immune checkpoint protein" has its general meaning in the art and refers to a molecule that is expressed by T cells in that either turn up a signal (stimulatory checkpoint molecules) or turn down a signal (inhibitory checkpoint molecules). Immune checkpoint molecules are recognized in the art to constitute immune checkpoint pathways similar to the CTLA-4 and PD-1 dependent pathways (see e.g. Pardoll, 2012. Nature Rev Cancer 12:252-264; Mellman et al. , 2011. Nature 480:480- 489). Examples of inhibitory checkpoint molecules include A2AR, B7-H3, B7-H4, BTLA, CTLA-4, CD277, IDO, KIR, PD-1, LAG-3, TIM-3 and VISTA. Inhibition includes reduction of function and full blockade. Preferred immune checkpoint inhibitors are antibodies that specifically recognize immune checkpoint proteins. A number of immune checkpoint inhibitors are known and in analogy of these known immune checkpoint protein inhibitors, alternative immune checkpoint inhibitors may be developed in the (near) future. The immune checkpoint inhibitors include peptides, antibodies, nucleic acid molecules and small molecules. Examples of immune checkpoint inhibitor includes PD-1 antagonist, PD-L1 antagonist, PD-L2 antagonist CTLA-4 antagonist, VISTA antagonist, TIM-3 antagonist, LAG-3 antagonist, IDO antagonist, KIR2D antagonist, A2AR antagonist, B7-H3 antagonist, B7-H4 antagonist, and BTLA antagonist.

In some embodiments, PD-1 (Programmed Death-1) axis antagonists include PD-1 antagonist (for example anti-PD-1 antibody), PD-L1 (Programmed Death Ligand-1) antagonist (for example anti-PD-L1 antibody) and PD-L2 (Programmed Death Ligand-2) antagonist (for example anti-PD-L2 antibody). In some embodiments, the anti-PD-1 antibody is selected from the group consisting of MDX-1106 (also known as Nivolumab, MDX-1106-04, ONO-4538, BMS-936558, and Opdivo®), Merck 3475 (also known as Pembrolizumab, MK-3475, Lambrolizumab, Keytruda®, and SCH-900475), and CT-011 (also known as Pidilizumab, hBAT, and hBAT-1). In some embodiments, the PD-1 binding antagonist is AMP-224 (also known as B7-DCIg). In some embodiments, the anti-PD-L1 antibody is selected from the group consisting of YW243.55.S70, MPDL3280A, MDX-1105, and MEDI4736. MDX-1105, also known as BMS-936559, is an anti-PD-L1 antibody described in WO2007/005874. Antibody YW243.55. S70 is an anti-PD-L1 described in WO 2010/077634 A1. MEDI4736 is an anti-PD-L1 antibody described in WO2011/066389 and US2013/034559. MDX-1106, also known as MDX-1106-04, ONO-4538 or BMS-936558, is an anti-PD-1 antibody described in U.S. Pat. No. 8,008,449 and WO2006/121168. Merck 3745, also known as MK-3475 or SCH-900475, is an anti-PD-1 antibody described in U.S. Pat. No. 8,345,509 and WO2009/114335. CT-011 (Pidizilumab), also known as hBAT or hBAT-1, is an anti-PD-1 antibody described in WO2009/101611. AMP-224, also known as B7-DCIg, is a PD-L2-Fc fusion soluble receptor described in WO2010/027827 and WO2011/066342. Atezolimumab is an anti-PD-L1 antibody described in U.S. Pat. No. 8,217,149. Avelumab is an anti-PD-L1 antibody described in US 20140341917. CA-170 is a PD-1 antagonist described in WO2015033301 & WO2015033299. Other anti-PD-1 antibodies are disclosed in U.S. Pat. No. 8,609,089, US 2010028330, and/or US 20120114649. In some embodiments, the PD-1 inhibitor is an anti-PD-1 antibody chosen from Nivolumab, Pembrolizumab or Pidilizumab. In some embodiments, PD-L1 antagonist is selected from the group comprising of Avelumab, BMS-936559, CA-170, Durvalumab, MCLA-145, SP142, STI-A1011, STIA1012, STI-A1010, STI-A1014, A110, KY1003 and Atezolimumab and the preferred one is Avelumab, Durvalumab or Atezolimumab.

In some embodiments, CTLA-4 (Cytotoxic T-Lymphocyte Antigen-4) antagonists are selected from the group consisting of anti-CTLA-4 antibodies, human anti-CTLA-4 antibodies, mouse anti-CTLA-4 antibodies, mammalian anti-CTLA-4 antibodies, humanized anti-CTLA-4 antibodies, monoclonal anti-CTLA-4 antibodies, polyclonal anti-CTLA-4 antibodies, chimeric anti-CTLA-4 antibodies, MDX-010 (Ipilimumab), Tremelimumab, anti-CD28 antibodies, anti-CTLA-4 adnectins, anti-CTLA-4 domain antibodies, single chain anti-CTLA-4 fragments, heavy chain anti-CTLA-4 fragments, light chain anti-CTLA-4 fragments, inhibitors of CTLA-4 that agonize the co-stimulatory pathway, the antibodies disclosed in PCT Publication No. WO 2001/014424, the antibodies disclosed in PCT Publication No. WO 2004/035607, the antibodies disclosed in U.S. Publication No. 2005/0201994, and the antibodies disclosed in granted European Patent No. EP 1212422 B. Additional CTLA-4 antibodies are described in U.S. Pat. Nos. 5,811,097; 5,855,887; 6,051,227; and 6,984,720; in PCT Publication Nos. WO 01/14424 and WO 00/37504; and in U.S. Publication Nos. 2002/0039581 and 2002/086014. Other anti-CTLA-4 antibodies that can be used in a method of the present invention include, for example, those disclosed in: WO 98/42752; U.S. Pat. Nos. 6,682,736 and 6,207,156; Hurwitz et al., Proc. Natl. Acad. Sci. USA, 95(17): 10067-10071 (1998); Camacho et al., J. Clin: Oncology, 22(145): Abstract No. 2505 (2004) (antibody CP-675206); Mokyr et al., Cancer Res., 58:5301-5304 (1998), and U.S. Pat. Nos. 5,977,318, 6,682,736, 7,109,003, and 7,132,281. A preferred clinical CTLA-4 antibody is human monoclonal antibody (also referred to as MDX-010 and Ipilimumab with CAS No. 477202-00-9 and available from Medarex, Inc., Bloomsbury, N.J.) is disclosed in WO 01/14424. With regard to CTLA-4 antagonist (antibodies), these are known and include Tremelimumab (CP-675,206) and Ipilimumab.

Other immune-checkpoint inhibitors include lymphocyte activation gene-3 (LAG-3) inhibitors, such as IMP321, a soluble Ig fusion protein (Brignone et al., 2007, J. Immunol. 179:4202-4211). Other immune-checkpoint inhibitors include B7 inhibitors, such as B7-H3 and B7-H4 inhibitors. In particular, the anti-B7-H3 antibody MGA271 (Loo et al., 2012, Clin. Cancer Res. July 15 (18) 3834). Also included are TIM-3 (T-cell immunoglobulin domain and mucin domain 3) inhibitors (Fourcade et al., 2010, J. Exp. Med. 207:2175-86 and Sakuishi et al., 2010, J. Exp. Med. 207:2187-94). As used herein, the term "TIM-3" has its general meaning in the art and refers to T cell immunoglobulin and mucin domain-containing molecule 3. The natural ligand of TIM-3 is galectin 9 (Gal9). Accordingly, the term "TIM-3 inhibitor" as used herein refers to a compound, substance or composition that can inhibit the function of TIM-3. For example, the inhibitor can inhibit the expression or activity of TIM-3, modulate or block the TIM-3 signaling pathway and/or block the binding of TIM-3 to galectin-9. Antibodies having specificity for TIM-3 are well known in the art and typically those described in WO2011155607, WO2013006490 and WO2010117057.

In some embodiments, the immune checkpoint inhibitor is an IDO inhibitor. Examples of IDO inhibitors are described in WO 2014150677. Examples of IDO inhibitors include without limitation 1-methyl-tryptophan (IMT), β- (3-benzofuranyl)-alanine, β-(3-benzo(b)thienyl)-alanine), 6-nitro-tryptophan, 6- fluoro-tryptophan, 4-methyl-tryptophan, 5 - methyl tryptophan, 6-methyl-tryptophan, 5-methoxy-tryptophan, 5 -hydroxy-tryptophan, indole 3-carbinol, 3,3'- diindolylmethane, epigallocatechin gallate, 5-Br-4-Cl-indoxyl 1,3-diacetate, 9- vinylcarbazole, acemetacin, 5-bromo-tryptophan, 5-bromoindoxyl diacetate, 3-Amino-naphtoic acid, pyrrolidine dithiocarbamate, 4-phenylimidazole a brassinin derivative, a thiohydantoin derivative, a β-carboline derivative or a brassilexin derivative. Preferably the IDO inhibitor is selected from 1-methyl-tryptophan, β-(3- benzofuranyl)-alanine, 6-nitro-L-tryptophan, 3-Amino-naphtoic acid and β-[3- benzo(b)thienyl] -alanine or a derivative or prodrug thereof.

In some embodiments, the second agent is an agent that induces, via ADCC, the death a cell expressing an antigen to which the second agent binds. In some embodiments, the agent is an antibody (e.g. of IgG1 or IgG3 isotype) whose mode of action involves induction of ADCC toward a cell to which the antibody binds. NK cells have an important role in inducing ADCC and increased reactivity of NK cells can be directed to target cells through use of such a second agent. In some embodiments, the second agent is an antibody specific for a cell surface antigens, e.g., membrane antigens. In some embodiments, the second antibody is specific for a tumor antigen as described above (*e.g*., molecules specifically expressed by tumor cells), such as CD20, CD52, ErbB2 (or HER2/Neu), CD33, CD22, CD25, MUC-1, CEA, KDR, αVβ3, etc., particularly lymphoma antigens (*e.g*., CD20). Accordingly, the present invention also provides methods to enhance the anti-tumor effect of monoclonal antibodies directed against tumor antigen(s). In the methods of the invention, ADCC function is specifically augmented, which in turn enhances target cell killing, by sequential administration of an antibody directed against one or more tumor antigens, and a compound of the present invention.

Accordingly, a further object relates to a method of enhancing NK cell antibody-dependent cellular cytotoxicity (ADCC) of an antibody in a subject in need thereof comprising administering to the subject the antibody, and administering to the subject a compound capable of stimulating CD28H on NK cells.

A further object of the present invention relates to a method of treating cancer in a subject in need thereof comprising administering to the subject a first antibody selective for a cancer cell antigen, and administering to the subject a compound capable of stimulating CD28H on NK cells.

A number of antibodies are currently in clinical use for the treatment of cancer, and others are in varying stages of clinical development. Antibodies of interest for the methods of the invention act through ADCC, and are typically selective for tumor cells, although one of skill in the art will recognize that some clinically useful antibodies do act on non-tumor cells, e.g. CD20. There are a number of antigens and corresponding monoclonal antibodies for the treatment of B cell malignancies. One popular target antigen is CD20, which is found on B cell malignancies. Rituximab is a chimeric unconjugated monoclonal antibody directed at the CD20 antigen. CD20 has an important functional role in B cell activation, proliferation, and differentiation. The CD52 antigen is targeted by the monoclonal antibody alemtuzumab, which is indicated for treatment of chronic lymphocytic leukemia. CD22 is targeted by a number of antibodies, and has recently demonstrated efficacy combined with toxin in chemotherapy-resistant hairy cell leukemia. Monoclonal antibodies targeting CD20, also include tositumomab and ibritumomab. Monoclonal antibodies useful in the methods of the invention, which have been used in solid tumors, include without limitation edrecolomab and trastuzumab (herceptin). Edrecolomab targets the 17-1 A antigen seen in colon and rectal cancer, and has been approved for use in Europe for these indications. Its antitumor effects are mediated through ADCC, CDC, and the induction of an anti-idiotypic network. Trastuzumab targets the HER- 2/neu antigen. This antigen is seen on 25% to 35% of breast cancers. Trastuzumab is thought to work in a variety of ways: downregulation of HER-2 receptor expression, inhibition of proliferation of human tumor cells that overexpress HER-2 protein, enhancing immune recruitment and ADCC against tumor cells that overexpress HER-2 protein, and downregulation of angiogenesis factors. Alemtuzumab (Campath) is used in the treatment of chronic lymphocytic leukemia; colon cancer and lung cancer; Gemtuzumab (Mylotarg) finds use in the treatment of acute myelogenous leukemia; Ibritumomab (Zevalin) finds use in the treatment of non-Hodgkin's lymphoma; Panitumumab (Vectibix) finds use in the treatment of colon cancer. Cetuximab (Erbitux) is also of interest for use in the methods of the invention. The antibody binds to the EGF receptor (EGFR), and has been used in the treatment of solid tumors including colon cancer and squamous cell carcinoma of the head and neck (SCCHN).

Typically, the compound of the present invention is administered to the subject in the form of a pharmaceutical composition which comprises a pharmaceutically acceptable carrier. Pharmaceutically acceptable carriers that may be used in these compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene- block polymers, polyethylene glycol and wool fat. For use in administration to a patient, the composition will be formulated for administration to the patient. The compositions of the present invention may be administered orally, parenterally, by inhalation spray, topically, rectally, nasally, buccally, vaginally or via an implanted reservoir. The used herein includes subcutaneous, intravenous, intramuscular, intra-articular, intra-synovial, intrasternal, intrathecal, intrahepatic, intralesional and intracranial injection or infusion techniques. Sterile injectable forms of the compositions of this invention may be aqueous or an oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono-or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation. The compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include, e.g., lactose. When aqueous suspensions are required for oral use, the active ingredient is combined with emulsifying and suspending agents. If desired, certain sweetening, flavoring or coloring agents may also be added. Alternatively, the compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols. The compositions of this invention may also be administered topically, especially when the target of treatment includes areas or organs readily accessible by topical application, including diseases of the eye, the skin, or the lower intestinal tract. Suitable topical formulations are readily prepared for each of these areas or organs. For topical applications, the compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water. Topical application for the lower intestinal tract can be effected in a rectal suppository formulation (see above) or in a suitable enema formulation. Patches may also be used. The compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents. For example, an antibody present in a pharmaceutical composition of this invention can be supplied at a concentration of 10 mg/mL in either 100 mg (10 mL) or 500 mg (50 mL) single-use vials. The product is formulated for IV administration in 9.0 mg/mL sodium chloride, 7.35 mg/mL sodium citrate dihydrate, 0.7 mg/mL polysorbate 80, and Sterile Water for Injection. The pH is adjusted to 6.5. An exemplary suitable dosage range for an antibody in a pharmaceutical composition of this invention may between about 1 mg/m² and 500 mg/m². However, it will be appreciated that these schedules are exemplary and that an optimal schedule and regimen can be adapted taking into account the affinity and tolerability of the particular antibody in the pharmaceutical composition that must be determined in clinical trials. A pharmaceutical composition of the invention for injection (e.g., intramuscular, i.v.) could be prepared to contain sterile buffered water (e.g. 1 ml for intramuscular), and between about 1 ng to about 100 mg, e.g. about 50 ng to about 30 mg or more preferably, about 5 mg to about 25 mg, of an anti-CD28H antibody of the invention.

The invention will be further illustrated by the following figures and examples. However, these examples and figures should not be interpreted in any way as limiting the scope of the present invention.

### FIGURES:

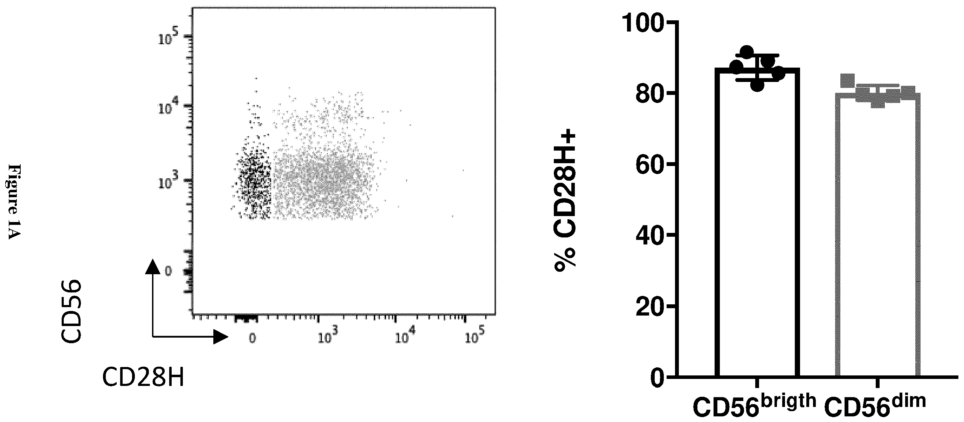

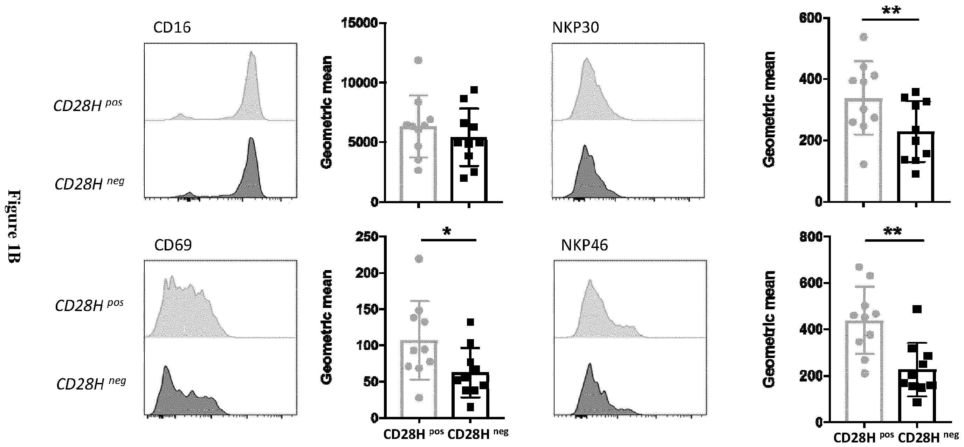

Figure 1: Circulating NK cells express CD28H. A detailed FACS analysis of CD28H^{pos} and CD28H^{neg} NK cells is shown. A, CD28H expression is sown on CD3^{neg}CD56^{bright} and CD3^{neg}CD56^{dim} NK cell populations. B, a detailed phenotype of CD28H^{pos} and CD28H^{neg} NK cells is shown.

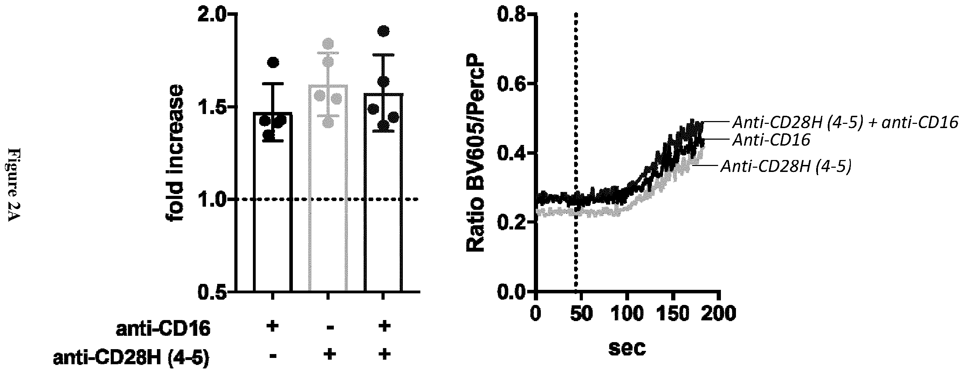

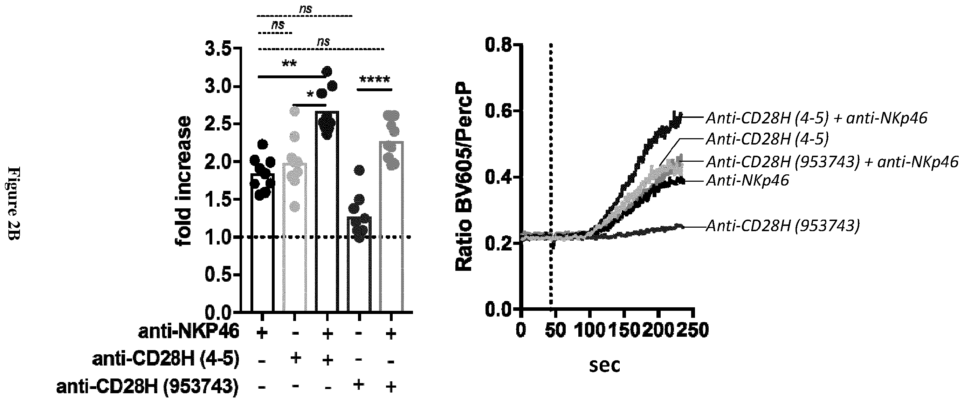

Figure 2: Activation of Ca2+ flux in resting NK cells by the anti-CD28H (clone 4-5). A, Ca2+ flux in resting NK cells is analyzed when either anti-CD28H (clone 4-5), anti-CD16 or both mAb are added on Fura Red-labeled NK cells. **B**, Ca2+ flux in resting NK cells is analyzed when the agonistic or the blocking anti-CD28H mAb are added on Fura Red-labeled NK cells with or without anti-NKp46. **A-B**, fold increase between the BV605/PercP ratios at 20sec and 180sec are plotted on the left, each symbol represents a specific experiment. On the right, two representative experiments of the BV605/PercP ratios are plotted as a function of time.

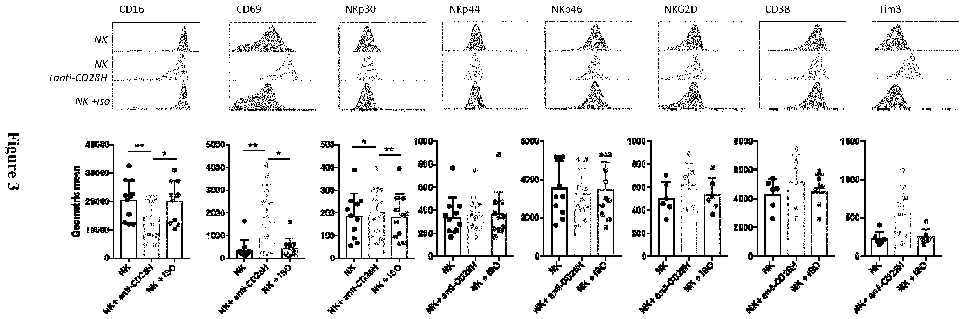

**Figure 3****:** Overnight triggering of CD28H activates NK cells. Freshly isolated NK cells cultured overnight with or without anti-CD28H or isotype control were then phenotype by FACS. A representative histogram of each activation marker is shown on the upper line, geometric means of independent experiments are plotted on the lower line.

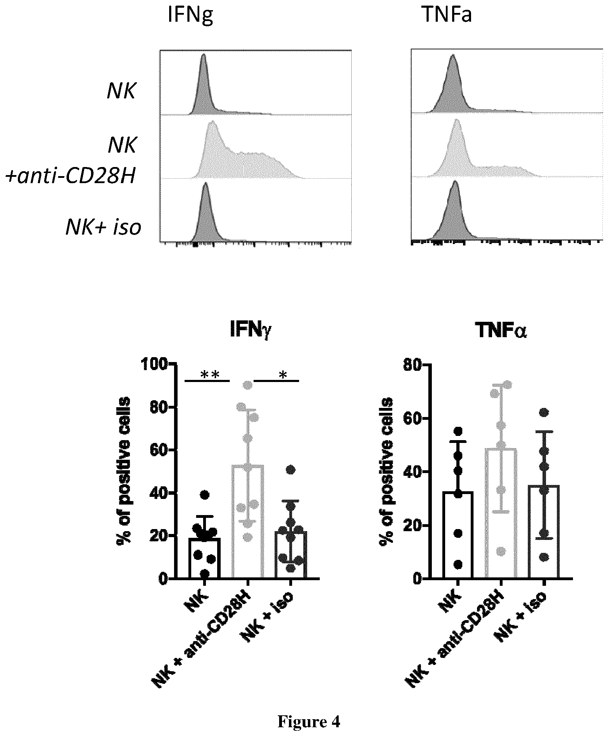

**Figure 4****:** CD28H-primed NK cells have increased helper function. Representative histograms of IFNg and TNFa intracellular expression of overnight CD28H-activated NK cells cultured the next day during 4h with K562 are shown on the upper line, geometric means of independent experiments are plotted on the lower line.

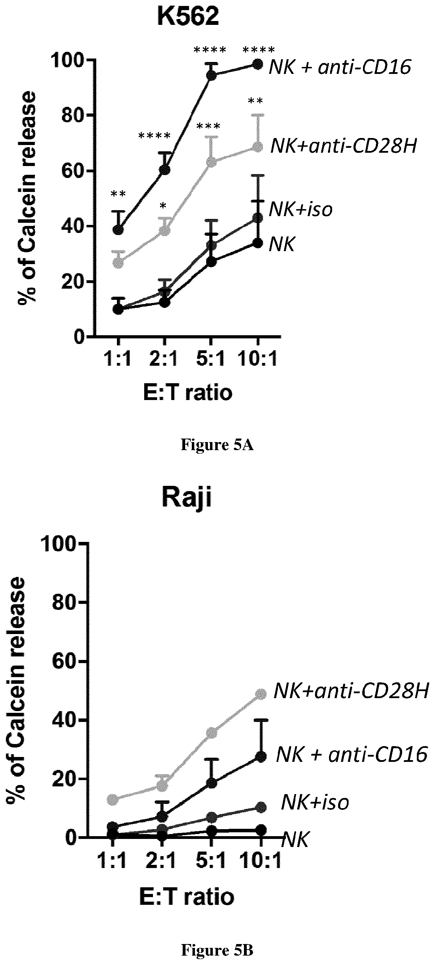

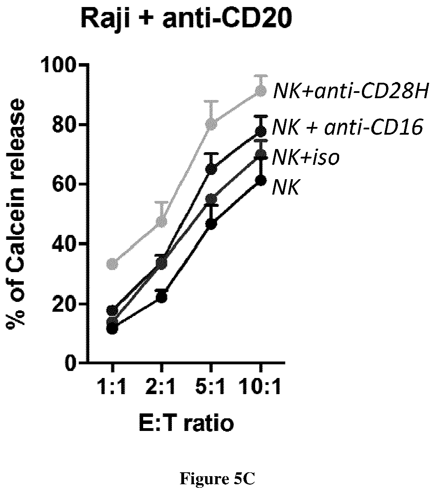

**Figure 5****:** CD28H-primed NK cells have increased direct and antibody-induced cytotoxicity to tumor cells. **A**, Engaging CD28H enhances NK cell killing of K562 tumor cells. **B**, CD28-H primed NK cells surpass CD16-primed NK cell in killing Raji target cells. C, CD28-H primed NK cells surpass CD16-primed NK cell in killing Raji target cells in an ADCC dependent manner.

### EXAMPLE:

**Objective** The main objective of this study is to evaluate the consequences of CD28H stimulation on NK with a monoclonal anti-CD28H antibody (clone 4-5) known to activate T cells.

### Methods :

### Cells

PBMC were isolated from cytapheresis ring from healthy donors (EFS, Nantes). Human NK cells were isolated by negative selection with the Mylteni NK cells isolation kit (ref 130-092-657). Tumor cells lines K562 (ATCC® CCL-243™), RAJI (ATCC® CCL-86™) were cultivated in complete RPMI medium (10% GE Healthcare HyClone™ Fetal Bovine Serum (U.S.), 1% Penicillin-Streptomycin Gibco®, 1% Glutamine) at a density of 5x10⁵ cells/ml, fresh medium were added every 2 days.

### Production of the anti-CD28H mAb clone 4-5

The hamster anti-human CD28H mAb (clone 4-5), generously provided by Dr Lieping Cheng, was cultured in RPMI with 10% fetal bovine serum (FBS). Antibodies in supernatant were purified by Hitrap protein G affinity column (GE Healthcare).

### Staining procedure and flow cytometry assays

Cells (5x10 ⁵ per well) suspended in 200µL of FACS buffer (Phosphate buffer Saline 1X, 1% Bovine serum albumin Sigma-Aldrich, 0,1% Azide Sigma-Aldrich) were plated in a V-bottom 96-well plate prior to be centrifuged at 2500 rpm 1min30. For extracellular staining, antibodies were added to each wells and cells were incubated for 30 minutes at 4°C in the dark. Finally, the cells were washed twice before fluorescence acquisition on a Celesta (BD Biosciences). Data were analyzed with FlowJo Software. For intracellular labelling, the BD Cytofix/Cytoperm kit was used according to the manufacture's protocol with recommended volume of intracellular antibodies.

The following anti-human antibodies were used: Anti-CD3 BUV395 (UCHT1, BD Biosciences,563546), Anti-CD56 PE (MY31, BD Biosciences, 556647), Anti-CD16 V450, (3G8, BD Biosciences, 561246), Anti-NKp30 BV711 (P30-15, BD Biosciences, 563383), Anti-NKp44 BV605 (P44-8, BD Biosciences, 744301), Anti-NKp46 PE-cy7 (9E2, BD Biosciences, 562101), Anti-CD69 Percpcy5.5 (FN50, BD Biosciences, 560738), Anti-Tim3 BV711 (7D3, BD Biosciences, 565566), anti-IFNy V450 (B27, BD Biosciences, 560371), anti-TNFa PE (Mab11, Biolegend, 502909), Anti-CD28H (953743, R&D, MAB83162), , Anti-CD28H (4-5, lab production) and the Anti-CD16 (3G8, lab production). The mouse Anti-CTLA-4 (UC10-4F10-11, lab production) was used as the isotype control of the anti-CD28H (4-5), both are Armenian Hamster Abs.

### Calcein cytotoxicity assays:

Freshly isolated NK cells were incubated overnight at 37°C (5% C02) in complete RPMI Medium supplemented with 10UI rhIL-2 (Cellgenix) and stimulated or not with 10ug/ml with the anti-CD28H (clone 4/5) or the anti-CD16 (clone 3G8) or the isotype control. After the overnight incubation NK cells are washed in PBS and resuspended at a density of 2x10⁶ cells/ml in complete medium RPMI. Five million of target cells were resuspended in 1ml of complete medium RPMI and incubated with 2 mM of Calcein, AM, cell-permeant dye (Invitrogen C3100MP) for 30min at 37°C (5% C02) in the dark. When mentioned, Rituximab (10ug/ml) was added during 30min on calcein-labeled cells. Cells were washed 3 times and resuspended at 2x10⁵ cells/ml in complete RPMI medium containing 10 mM of Probenecid (Invitrogen, P36400). NK cells and target cells were incubated for 4h at 30min at 37°C (5% C02) with 20,000 labelled tumor cells at different Effector:Target ratios i.e. 10:1 (200 000 / 20 000) ; 5:1 (100 000 / 20 000) ; 2:1 (40 000 / 20 000) and 1:1 (20 000/ 20 000). After the incubation, the wells were gently mixed to uniformly distribute the released calcein in the supernatant. The plates were then centrifuged for 2 minutes at 2000 rpm. One hundred microliter of supernatant were recovered into black 96-well plates (Greiner, 655076) and the fluorescence level was analyzed with at Spark® plate reader (TECAN) with excitation and emission at 470nm and 509nm, respectively. Maximum and spontaneous release controls were set up in duplicate using 1% Triton X-100 Sigma-Aldrich and RPMI complete medium, respectively. Specific lysis was calculated using the formula [(Test release-Spontaneous release) / (Maximum release-Spontaneous release)] × 100.

### Calcium flux:

One million NK cells were incubated with 5µM of Fura Red™, AM, cell permeant (F3020) in 200µl of calcium-free HBSS medium for 30 min at 37°C. Then, NK cells were incubated on ice for 30 min with primaries antibodies. Cells were resuspended in 500µL of HBSS supplemented with 10mM HEPES, 1% BSA, 1mM CaCl₂, acquired during 30 sec with the FACS Celesta (BD Biosciences) prior to the addition of a cross-linker mix to induce calcium flow. The cross-linker mix was composed of 4ug of the anti-Armenian hamster (Jackson, 127-545-16) and 4ug a goat anti-mouse F(ab')₂(Jackson Immuno Research), samples were vortexed and acquired on the FACS Celesta for at least 4 min. Data were analyzed with FlowJo, cells were gated and the mean fluorescence intensity of the BV605 and the Percp was calculated and plotted as a function of time.

### Results

CD28H is widely expressed on peripheral human NK cells, as shown in Figure 1. The expression of CD28H by CD56^{bright} NK cells and NK CD56^{dim} cells is not different (Figure 1A). CD28H⁺ NK cells and CD28H^{neg} NK cells were further analyzed for NK cell specific markers; both populations similarly express CD16, whereas CD28H^{pos} NK cells express significantly more CD69, NKP30 and NKP46 (Figure 1B), suggesting that CD28H^{pos} NK cells would be more activated than CD28H^{neg} NK cells.

Zhuang et al have described that the anti-CD28H blocking (clone 953743, R&D) was not able to induce by itself a reverse-ADCC using P815 cells. On the other hand, a synergistic effect was observed between the engagement of CD28H and CD16, CD28H and 2B4 and, to a lesser extent, between the co-engagement of CD28H and NKP46. Since the antibody (clone 953743, R&D) blocks the CD28H-HHLA2 interaction and is a mouse antibody, we wondered if the clone used induces the activation of targeted NK cell or if it is a "passive" reverse-ADCC due for example to the clustering of CD28H on the surface of NK cells. Some NK cell activating receptors, targeted by specific mAb, may directly generate Ca²⁺ influx, while others only induces Ca²⁺ flux in a synergistic manner (Bryceson: 2006c). Freshly isolated NK cells, labeled with a Fura RedTM probe, were incubated with the anti-CD28H clone 4-5, an anti-CD16 (clone 3G8) or both antibodies together and analyzed by FACS. The anti-CD28H clone 4-5 does not induce a calcium flux alone while the anti-CD16 slightly activates NK cells. The primary antibodies were then crosslinked by the addition of a cross-linker mixt and the calcium flux was measured by FACS during 4min. The anti-CD28H (clone 4-5) induces a calcium flux as important as that induced by the anti-CD16, no additive effect was observed when NK cells were incubated with these two antibodies (Figure 2A). Conversely, whereas an anti-NKP46 and the anti-CD28H (clone 4-5) induce a similar calcium flux when the cross-linker mixt was added, a significantly higher calcium flux was observed when the anti-NKP46 and the anti-CD28H clone 4-5 were added together on the same NK cells. Interestingly, the blocking anti-CD28H (clone 953743, R&D) does not induce a calcium flux even after the addition of the cross-linker mixt and no synergistic effect was observed with the anti-NKP46 (Figure 2B).

Freshly isolated NK cells from healthy volunteers were stimulated over night with the anti-CD28H (clone 4-5), a control isotype (mouse anti-CTLA4, clone 4F10) or an anti-CD16, in the presence of 10U/ml of hr-IL2. The next day, the expression of different activation markers was analyzed by FACS. NK cells stimulated overnight with the anti-CD28H express significantly less CD16 but more CD69 and NKP30. Tim3, CD38 and NKG2D also appear to be more expressed when CD28H is stimulated (Figure 3) suggesting that stimulating CD28H with the clone 4-5 results in NK cells activation.

Because the overnight-stimulation of NK cells with the anti-CD28H (clone4-5) results in their activation, such CD28H-activated NK cells were cultured during 4h with K562 and their helper function were analyzed by intracellular FACS staining of IFNg and TNFa. CD28H-activated NK cells showed increase expression of IFNg and TNFa when cultured with K562 tumor cells (Figure 4).

It was unknown whether cytotoxicity could be mediated by CD28H triggering (Figure 5). A classical calcein-released assay was setup. When overnight-CD28H-activated NK cells were used as effectors and K562 as target cells at different E:T ratio, an increased specific lysis was observed as compared to negative controls (NK cells and NK cells cultured overnight with the isotype) (Figure 5A). CD16-primed NK cells were used here as a positive control. Interestingly, overnight-CD28H-activated NK cells surpass overnight-CD16-primed NK cells in direct killing of Raji target cells (Figure 5B). Because direct cytotoxicity of CD28H-primed NK cells was increased, we also evaluated the antibody-induced cytotoxicity. Overnight-CD28H-activated NK cells surpass overnight-CD16-primed NK cells in antibody-opsonized Raji target cells.

### REFERENCES:

Throughout this application, various references describe the state of the art to which this invention pertains. The disclosures of these references are hereby incorporated by reference into the present disclosure.

## Claims

1. A method of enhancing NK cell killing activities in a subject in need thereof comprising administering to the subject a therapeutically effective amount of a compound capable of stimulating CD28H on NK cells.

2. The method of claim 1 wherein the compound capable of stimulating CD28H on NK cells is an antibody having specificity to CD28H.

3. The method of claim 2 wherein the antibody binds the extracellular domain of CD28H.

4. The method of claim 2 wherein the antibody binds to an epitope located in the extracellular domain of CD28H.

5. The method of claim 2 wherein the antibody is a chimeric, a humanized or a human antibody.

6. The method of claim 2 wherein the antibody cross-competes for binding to the CD28H isoform with the antibody comprising a light chain variable region comprising the following CDR: i) the VL-CDR1 as set forth in SEQ ID NO:2 (QSLFSSNTKRNY), ii) the VL-CDR2 as set forth in SEQ ID NO:3 (HAS) and iii) the VL-CDR3 as set forth in SEQ ID NO:4 (GFSITTGGYY) and a heavy chain variable region comprising the following CDR i) the VH-CDR1 as set forth in SEQ ID NO:5 (GYTFTSYW), ii) the VH-CDR2 as set forth in SEQ ID NO:6 (IYTSGRT) and iii) the VH-CDR3 as set forth in SEQ ID NO:7 (ADMADKGGWFAY).

7. The method of claim wherein the antibody cross-competes for binding to the CD28H isoform with the antibody comprising a light chain variable region as set forth in SEQ ID NO:8 and a heavy chain variable region as set forth in SEQ ID NO:9.

8. The method of claim 2 wherein the antibody comprises a light chain variable region comprising the following CDR: i) the VL-CDR1 as set forth in SEQ ID NO:2 (QSLFSSNTKRNY), ii) the VL-CDR2 as set forth in SEQ ID NO:3 (HAS) and iii) the VL-CDR3 as set forth in SEQ ID NO:4 (GFSITTGGYY) and a heavy chain variable region comprising the following CDR i) the VH-CDR1 as set forth in SEQ ID NO:5 (GYTFTSYW), ii) the VH-CDR2 as set forth in SEQ ID NO:6 (IYTSGRT) and iii) the VH-CDR3 as set forth in SEQ ID NO:7 (ADMADKGGWFAY).

9. The method of claim 2 wherein the antibody comprises a light chain variable region as set forth in SEQ ID NO:8 and/or a heavy chain variable region as set forth in SEQ ID NO:9.

10. The method of claim 1 wherein the subject suffers from a cancer or an infectious disease.

11. A method of treating a cancer or an infectious disease in a subject in need thereof comprising administering to the subject a therapeutically effective amount of a compound capable of stimulating CD28H on NK cells.

12. The method of claim 11 wherein the compound capable of stimulating CD28H on NK cells is administered to the patient in combination with an immune checkpoint inhibitor.

13. A method of enhancing NK cell antibody-dependent cellular cytotoxicity (ADCC) of an antibody in a subject in need thereof comprising administering to the subject the antibody, and administering to the subject a compound capable of stimulating CD28H on NK cells.

14. A method of treating cancer in a subject in need thereof comprising administering to the subject a first antibody selective for a cancer cell antigen, and administering to the subject a compound capable of stimulating CD28H on NK cells.


SEQUENCE LISTING

<110>  Inserm

<120>  CD28H STIMULATION ENHANCES NK CELL KILLING ACTIVITIES

<130>  BIO19076 HASPOT / MC

<160>  9

<170>  PatentIn version 3.3

<210>  1
<211>  282
<212>  PRT
<213>  Homo sapiens

<400>  1

Met Gly Ser Pro Gly Met Val Leu Gly Leu Leu Val Gln Ile Trp Ala
1               5                   10                  15

Leu Gln Glu Ala Ser Ser Leu Ser Val Gln Gln Gly Pro Asn Leu Leu
            20                  25                  30

Gln Val Arg Gln Gly Ser Gln Ala Thr Leu Val Cys Gln Val Asp Gln
        35                  40                  45

Ala Thr Ala Trp Glu Arg Leu Arg Val Lys Trp Thr Lys Asp Gly Ala
    50                  55                  60

Ile Leu Cys Gln Pro Tyr Ile Thr Asn Gly Ser Leu Ser Leu Gly Val
65                  70                  75                  80

Cys Gly Pro Gln Gly Arg Leu Ser Trp Gln Ala Pro Ser His Leu Thr
                85                  90                  95

Leu Gln Leu Asp Pro Val Ser Leu Asn His Ser Gly Ala Tyr Val Cys
            100                 105                 110

Trp Ala Ala Val Glu Ile Pro Glu Leu Glu Glu Ala Glu Gly Asn Ile
        115                 120                 125

Thr Arg Leu Phe Val Asp Pro Asp Asp Pro Thr Gln Asn Arg Asn Arg
    130                 135                 140

Ile Ala Ser Phe Pro Gly Phe Leu Phe Val Leu Leu Gly Val Gly Ser
145                 150                 155                 160

Met Gly Val Ala Ala Ile Val Trp Gly Ala Trp Phe Trp Gly Arg Arg
                165                 170                 175

Ser Cys Gln Gln Arg Asp Ser Gly Asn Ser Pro Gly Asn Ala Phe Tyr
            180                 185                 190

Ser Asn Val Leu Tyr Arg Pro Arg Gly Ala Pro Lys Lys Ser Glu Asp
        195                 200                 205

Cys Ser Gly Glu Gly Lys Asp Gln Arg Gly Gln Ser Ile Tyr Ser Thr
    210                 215                 220

Ser Phe Pro Gln Pro Ala Pro Arg Gln Pro His Leu Ala Ser Arg Pro
225                 230                 235                 240

Cys Pro Ser Pro Arg Pro Cys Pro Ser Pro Arg Pro Gly His Pro Val
                245                 250                 255

Ser Met Val Arg Val Ser Pro Arg Pro Ser Pro Thr Gln Gln Pro Arg
            260                 265                 270

Pro Lys Gly Phe Pro Lys Val Gly Glu Glu
        275                 280

<210>  2
<211>  12
<212>  PRT
<213>  Mus musculus

<400>  2

Gln Ser Leu Phe Ser Ser Asn Thr Lys Arg Asn Tyr
1               5                   10

<210>  3
<211>  3
<212>  PRT
<213>  Mus musculus

<400>  3

His Ala Ser
1

<210>  4
<211>  10
<212>  PRT
<213>  Mus musculus

<400>  4

Gly Phe Ser Ile Thr Thr Gly Gly Tyr Tyr
1               5                   10

<210>  5
<211>  8

<212>  PRT
<213>  Mus musculus

<400>  5

Gly Tyr Thr Phe Thr Ser Tyr Trp
1               5


<210>  6
<211>  7
<212>  PRT
<213>  Mus musculus

<400>  6

Ile Tyr Thr Ser Gly Arg Thr
1               5


<210>  7
<211>  12
<212>  PRT
<213>  Mus musculus

<400>  7

Ala Asp Met Ala Asp Lys Gly Gly Trp Phe Ala Tyr
1               5                   10


<210>  8
<211>  113
<212>  PRT
<213>  Mus musculus

<400>  8

Asp Ile Val Met Thr Gln Ser Pro Ser Ser Leu Ala Val Ser Ala Gly
1               5                   10                  15


Glu Lys Val Thr Ile Ser Cys Leu Ser Ser Gln Ser Leu Phe Ser Ser
            20                  25                  30


Asn Thr Lys Arg Asn Tyr Leu Asn Trp Tyr Leu Gln Lys Pro Gly Gln
        35                  40                  45


Ser Pro Lys Leu Leu Ile Tyr His Ala Ser Thr Arg Leu Thr Gly Val
    50                  55                  60


Pro Gly Arg Phe Ile Gly Ser Gly Ser Gly Thr Asp Phe Thr Leu Thr
65                  70                  75                  80


Val Ser Thr Val Gln Ala Glu Asp Leu Gly Asp Tyr Phe Cys Gln Gln
                85                  90                  95


His Tyr Glu Thr Pro Leu Thr Phe Gly Asp Gly Thr Arg Leu Glu Ile

            100                 105                 110

Lys

<210> 9
<211> 120
<212> PRT
<213> Mus musculus

<400> 9

Gln Ile Gln Leu Gln Glu Ser Gly Pro Gly Leu Val Lys Pro Ser Gln
1               5                   10                  15

Ser Leu Ser Leu Thr Cys Ser Val Thr Gly Phe Ser Ile Thr Thr Gly
            20                  25                  30

Gly Tyr Tyr Trp Asn Trp Ile Arg Gln Phe Pro Gly Lys Lys Leu Glu
        35                  40                  45

Trp Met Gly Tyr Ile Tyr Thr Ser Gly Arg Thr Ser Tyr Asn Pro Ser
    50                  55                  60

Leu Lys Ser Arg Ile Ser Ile Thr Arg Asp Thr Ser Lys Asn Gln Phe
65                  70                  75                  80

Phe Leu Gln Leu Asn Ser Met Thr Thr Glu Asp Thr Ala Thr Tyr Tyr
                85                  90                  95

Cys Ala Asp Met Ala Asp Lys Gly Gly Trp Phe Ala Tyr Trp Gly Gln
            100                 105                 110

Gly Thr Leu Val Thr Val Ser Ser
        115                 120